# EUROPEAN PATENT SPECIFICATION

(11) **EP 2 972 326 B1**
(45) Date of publication and mention of the grant of the patent: **08.01.2020**
(21) Application number: 14721649.3
(22) Date of filing: 14.03.2014
(51) Int. Cl.: G01N 33/50, G01N 33/68, G01N 33/94

(54) **A NONINVASIVE METHOD FOR MEASURING METABOLITES FOR SKIN HEALTH**
NICHT-INVASIVES VERFAHREN ZUR MESSUNG VON METABOLITEN FÜR DIE HAUTGESUNDHEIT
MÉTHODE NON INVASIVE POUR MESURER DES MÉTABOLITES POUR LA SANTÉ DE LA PEAU

(30) Priority: 15.03.2013 US 201361793719 P
(43) Date of publication of application: 20.01.2016
(73) Proprietor: The Procter & Gamble Company, Cincinnati, OH 45202 (US)
(72) Inventor: KERR, Kathleen Marie, Cincinnati, Ohio 45202 (US); WEHMEYER, Kenneth Robert, Cincinnati, Ohio 45202 (US); WANG, Feng, Cincinnati, Ohio 45202 (US); FIENO, Angela Marie, Cincinnati, Ohio 45202 (US); MILLS, Kevin John, Cincinnati, Ohio 45202 (US)
(74) Representative: P&G Patent Germany
(86) International application number: PCT/US2014/028537
(87) International publication number: WO 2014/144223

(56) References cited:
- WO-A1-2007/096846
- WO-A1-2007/096846
- WO-A1-2011/087523
- US-A1- 2008 261 317
- US-A1- 2008 261 317
- US-A1- 2009 318 556
- US-A1- 2009 318 556
- US-A1- 2011 071 123
- PRABHAKARAN K ET AL: "Glutamic acid decarboxylase activity of Mycobacterium leprae and occurrence of gamma-amino butyric acid in skin lesions of leprosy.", NATURE 10 NOV 1962, vol. 196, 10 November 1962 (1962-11-10), pages 589-590, XP002763301, ISSN: 0028-0836
- ROSSOMANDO E F ET AL: "ALTERATIONS IN PURINE SALVAGE AND HYPO XANTHINE LEVELS IN GRANULATION TISSUE DURING SKIN WOUND REPAIR", JOURNAL OF SURGICAL RESEARCH, vol. 35, no. 3, 1983, pages 259-263, XP055312711, ISSN: 0022-4804
- HU ZE-PING ET AL: "Metabolomic response of human skin tissue to low dose ionizing radiation", MOLECULAR BIOSYSTEMS, vol. 8, no. 7, 2012, pages 1979-1986, XP002727586,
- RANDHAWA MANPREET ET AL: "Metabolomic analysis of sun exposed skin.", MOLECULAR BIOSYSTEMS AUG 2013, vol. 9, no. 8, August 2013 (2013-08), pages 2045-2050, XP002727587, ISSN: 1742-2051
- PRABHAKARAN K ET AL: "Glutamic acid decarboxylase activity of Mycobacterium leprae and occurrence of gamma-amino butyric acid in skin lesions of leprosy.", NATURE 10 NOV 1962, vol. 196, 10 November 1962 (1962-11-10), pages 589-590, XP002763301, ISSN: 0028-0836
- ROSSOMANDO E F ET AL: "ALTERATIONS IN PURINE SALVAGE AND HYPO XANTHINE LEVELS IN GRANULATION TISSUE DURING SKIN WOUND REPAIR", JOURNAL OF SURGICAL RESEARCH, vol. 35, no. 3, 1983, pages 259-263, XP055312711, ISSN: 0022-4804

## Description

### FIELD OF THE INVENTION

The present invention relates to a method for measuring the amount of one or more small molecule and or metabolite biomarker from a skin sample and the link and correlation of the amount of these biomarkers as it directly correlates to improvement in dandruff in mammals. The biomarker is uridine.

### BACKGROUND OF THE INVENTION

The scalp/skin is a remarkable organ system composed of multiple specialized tissues that function as a first line of defense against environmental insults. While early research focused primarily on the barrier function of the skin, it has become clear that that this organ is dynamic: sensing and responding to even small changes in the environment in order to help maintain homeostasis. Environmental influences such as solar radiation, pollution, or even the application of skin care products, result in a complex cascade of events that ultimately lead to changes in the expression of hundreds or thousands of genes. These changes in gene expression are generally translated to changes in protein production (or accumulation, release, modification etc.) that catalyze chemical reactions ultimately leading to the cellular response. As these chemical reactions proceed, metabolic byproducts are often left as an indicator of what chemical processes have taken place. Profiling analysis of those small molecule biomarkers that correlate to a given skin condition, for example, dandruff can provide valuable information in understanding the condition as well as developing products for the purpose of diagnosing and/or improving the skin condition.

Dandruff is a common chronic relapsing scalp skin condition with flaking, itching and irritation as signs and symptoms. The pathogenesis of dandruff is complex, and appears to be the result of interactions among scalp skin, microflora and the host immune system. Much of the previous work on this condition has focused on the examination of a few surface-level phenomena. Traditional expert- and self-observation-based assessments are combined with largely instrumental-based assessments of epidermal structure and function at the physiological level. New biomolecular capabilities establish a depth of pathophysiological understanding not previously achievable with traditional means of investigation; however, a clear picture of the molecular events leading to the key symptoms of this condition has yet to emerge. To elucidate these key molecular events, biomolecular sampling can be obtained noninvasively by tape stripping of the skin surface followed by chemical or bioanalytical methodologies. Histamine was recently identified as a sensitive biomarker for scalp itch. Additional biomarkers are needed to enable a more detailed pathophysiological description of the dandruff condition as well as serve as relevant measures that are indicative of the extent and completeness of therapeutic resolution of dandruff. Biomarkers that can be sampled noninvasively, will enable the greatest utility for use in routine clinical evaluations.

In order to indentify biomarkers for dandruff condition, a metabolite profiling approach is used to distinguish the difference among scalp tape strip extracts from non dandruff, dandruff, and dandruff subjects treated with anti-dandruff shampoo. This metabolite profiling method (metabolomics) represents unique footprints of cellular processes for each subject group. While genomics and proteomics provide snapshots of what may be happening in a biological system, metabolomics can be regarded as the amplified output of biological systems in response to genetic and environmental changes, giving us valuable information on the physiology of the system. Successful integration of the metabonomic profile with genomics and proteomic understanding will enable a more complete understanding of biological systems including applications in dermatology.

Noninvasive sampling methods are important for biomarker applications in skin /scalp care. Tape strips have been successfully used for collection of small molecules from skin/scalp for subsequent metabolite profiling analyses.

US Patent Application No.: 2011/0071123 A1 describes a method for measuring of histamine in epidermis as an objective measurement of itch.

International Application WO 2011/087523 A1 discloses methods for selecting and formulating compositions for treating and maintaining the quality of skin, wherein a composition is selected for use in a personal care product based on its demonstrated biological effect to improve skin quality as evidenced by one or more biomarker changes that correlate with improvement as evidenced by one or more objective measurements of skill health.

Hu et al. study the human organ respond to ionizing radiation at a systems biology level and identify how biomarkers for IR exposure at low doses can help provide a scientific basis for establishing radiation protection standards (Mol. BioSyst., 2012, 8, 1979-1986).

Rossomando and Bertolami analyze alterations of purine salvage and hypoxanthine levels in granulation tissue during skin wound repair (Journal of Surgical Research, 983, 35, 259-263).

### SUMMARY OF THE INVENTION

The present invention is directed to a noninvasive method for diagnosing dandruff in a subject as defined by the appended claims.

### BRIEF DESCRIPTION OF THE TABLES

Table 1a, b, c, d, e. Fold change of metabolites with significant statistical difference between dandruff and nondandruff, and dandruff upon anti-dandruff treatments.
Table 2a and b. Amino acid biomarkers and their metabolites with significant differences.
Table 3a and b. Dipeptides metabolites/biomarkers with significant differences.
Table 4. Nucleic acid metabolites/biomarkers with significant differences.
Table 5a and b. Lipids biomarkers and their metabolites/biomarkers with significant differences.
Table 6. Miscellaneous biomarkers including carbohydrates and their metabolites, co-factors, and others with significant differences.
Table 7a and b. Structure unknown molecules/biomarkers with significant differences.
Table 8 Self Assessment and Expert Grading Results

### DETAILED DESCRIPTION OF THE INVENTION

While the specification concludes with claims which particularly point out and distinctly claim the invention, it is believed the present invention will be better understood from the following description.

The present invention can comprise, consist of, or consist essentially of the essential elements and limitations of the invention described herein, as well any of the additional or optional ingredients, components, or limitations described herein.

All percentages, parts and ratios are based upon the total weight of the compositions of the present invention, unless otherwise specified. All such weights as they pertain to listed ingredients are based on the active level and, therefore, do not include carriers or by-products that may be included in commercially available materials.

The components and/or steps, including those, which may optionally be added, of the various embodiments of the present invention, are described in detail below.

All ratios are weight ratios unless specifically stated otherwise.

All temperatures are in degrees Celsius, unless specifically stated otherwise.

Except as otherwise noted, all amounts including quantities, percentages, portions, and proportions, are understood to be modified by the word "about", and amounts are not intended to indicate significant digits.

Except as otherwise noted, the articles "a", "an", and "the" mean "one or more".

Herein, "comprising" means that other steps and other ingredients which do not affect the end result can be added. This term encompasses the terms "consisting of" and "consisting essentially of". The compositions and methods/processes of the present invention can comprise, consist of, and consist essentially of the essential elements and limitations of the invention described herein, as well as any of the additional or optional ingredients, components, steps, or limitations described herein.

Herein, "effective" means an amount of a subject active high enough to provide a significant positive modification of the condition to be treated. An effective amount of the subject active will vary with the particular condition being treated, the severity of the condition, the duration of the treatment, the nature of concurrent treatment, and like factors.

As used herein, the term "differential level" of a metabolite may include any increased or decreased level. In one embodiment, differential level means a level that is increased by: at least 5%; by at least 10%; by at least 20%; by at least 30%; by at least 40%; by at least 50%; by at least 60%; by at least 70%; by at least 80%; by at least 90%; by at least 100%; by at least 110%; by at least 120%; by at least 130%; by at least 140%; by at least 150%; or more. In another embodiment, differential level means a level that is decreased by: at least 5%; by at least 10%; by at least 20%; by at least 30%; by at least 40%; by at least 50%; by at least 60%; by at least 70%; by at least 80%; by at least 90%; by at least 100%. A metabolite is expressed at a differential level that is statistically significant (i.e., a p-value equal and less than 0.2 as determined using, either Student T-test, Welch's 2 sample T-test, Matched Pair T-test or Wilcoxon's rank-sum Test).

As used herein, the term "metabolite" means any substance produced by metabolism or necessary for or taking part in a particular metabolic process. The term does not include large macromolecules, such as large proteins (e.g, proteins with molecular weights over 2,000, 3,000, 4,000, 5,000, 6,000, 7,000, 8,000, 9,000, or 10,000); large nucleic acids (e.g., nucleic acids with molecular weights of over 2,000, 3,000, 4,000, 5,000, 6,000, 7,000, 8,000, 9,000, or 10,000); or large polysaccharides (e.g., polysaccharides with a molecular weights of over 2,000, 3,000, 4,000, 5,000, 6,000, 7,000, 8,000, 9,000, or 10,000). The term metabolite includes signaling molecules and intermediates in the chemical reactions that transform energy derived from food into usable forms including, but not limited to: sugars, fatty acids, amino acids, nucleotides, antioxidants, vitamins, co-factors, lipids, intermediates formed during cellular processes, and other small molecules.

In the present invention, the detected small molecule biomarker is uridine.

The term 'skin' means the outer covering of a vertebrate animal, consisting of two layers of cells, a thick inner layer (the dermis) and a thin outer layer (the epidermis). The epidermis is the external, nonvascular layer of the skin. It is made up, from within outward, of five layers of EPITHELIUM: (1) basal layer (stratum basale epidermidis); (2) spinous layer (stratum spinosum epidermidis); (3) granular layer (stratum granulosum epidermidis); (4) clear layer (stratum lucidum epidermidis); and (5) horny layer (stratum corneum epidermidis).

The term "sample" refers to any preparation from skin or epidermis of a subject.

The term "noninvasive" means a procedure that does not require insertion of an instrument or device through the skin or a body orifice for diagnosis or treatment.

The term "adhesive device" means a device used for the removal of the skin's epidermal layer by using an adhesive or an adhesive material on a substrate. For example, skin samples with adhesive tapes such as D-Squame® (polyacrylate ester adhesives; CuDerm; Dallas TX), Durapor, Sebutape™ (acrylic polymer films; CuDerm; Dallas, TX), Tegaderm™, Duct tape (333 Duct Tape, Nashua tape products), Scotch® Tape (3M Scotch 810, St. Paul, Minn.), Diamond™ (The Sellotape Company; Eindhoven, the Netherlands), Sentega™ (polypropylene tape, Sentega Eiketten BV, Utrecht, The Netherlands) may be used. The adhesive may be any of the commonly used pressure-sensitive-type adhesives or those which solidify quickly upon skin content (such as cynaoacylates). The adhesives may be on flexible or solid backings to make sampling easier. A constant pressure device (e.g. Desquame Pressure Instrument, CuDerm; Dallas, TX) can be used to apply pressure to the adhesive device during sampling.

Samples from a tissue may be isolated by any number of means well known in the art. Invasive methods for isolating a sample include the use of needles, for example during blood sampling, as well as biopsies of various tissues, blistering techniques and laser poration. Due to the invasive nature of these techniques there is an increased risk of mortality and morbidity. Further, invasive techniques can inadvertently impact the state of the skin, which could lead to inaccurate or false results. Even further, invasive techniques are difficult to execute on a large population. The invasive technique may result in discomfort to the participant and may provide a greater potential for infection or other side effects. The present invention provides a noninvasive method for measuring small molecules from the skin.

The term "objectively" means without bias or prejudice. Alternatively, any expert or self-assessments are inherently "subjective."

The term "normalization" and/or "normalized" means the degree to which a population of dandruff sufferers approaches a state of normal population.

The term "standardization" and/or "standardized" means small molecule values expressed relative to the amount of protein measured on the corresponding adhesive or adhesive article. A non-limiting example would be ng small molecule/ µg soluble protein.

The term "baseline" means information gathered at the beginning of a study from which variations found in the study are measured. A baseline sample may be from a dandruff sufferer or a non-dandruff sufferer.

In a further embodiment of the present invention, there are a number of alternative "Noninvasive" Sampling Methods that may be used.

Sebutape™: This is a noninvasive approach in that Sebutape™ (acrylic polymer film; CuDerm; Dallas, TX) is only very mildly adhesive and may be applied to and removed from even visibly inflamed skin without causing discomfort. Biomarkers recovered/assayed by this technique have included proteins (e.g., cytokines), peptides (e.g., neuropeptides), and small molecule (e.g., nitric oxide) mediators. Historically, this tape is manufactured and sold for sebum collection and can, therefore, be useful for lipid analysis.

D-Squame®: D-Squame® tape is a polyacrylate ester adhesive also manufactured by CuDerm. It may be used to recover the same biomarkers as Sebutape™ but also removes certain epidermal structural proteins (e.g., keratins, involucrin). Cup Scrubs: Cup scrubs extract proteins directly from the surface of the skin, usually in the presence of buffer and a nonionic surfactant. Cup scrubs are primarily used for recovery of soluble biomarkers such as cytokines, but can also be used to recover small organic molecules. Many more cytokines can be recovered and quantified from cup scrubs than from tape strips. This could be due to several reasons. (a) Due to the presence of detergents and their liquid nature, cup scrubs most likely sample a different protein population than do tape strips. (b) With cup scrubs, cytokines do not have to be further extracted after sample collection since they already are in solution.

Hair plucks: Plucking hairs is the process of removing human or animal hair by mechanically pulling the item from the owner's body usually with tweezers. The follicular region of the hair pluck is extracted usually in the presence of buffer and a nonionic surfactant for recovery of soluble biomarkers such as cytokines, and can also be extracted with an organic solvent to recover small organic molecules.

Animal (i.e. Dog) Collection Method: D-Squame®: D- Squame™ tape samples are collected on dogs' skin via parting their fur (without shaving). A variety of biomarkers related to skin inflammation, differentiation and barrier integrity can be analyzed from the tapes including total protein, soluble protein, skin multiple analyte profile (skin MAP), skin cytokines and stratum corneum lipids (ceramides, cholesterol, fatty acids).

Described herein is a method and analysis for noninvasively obtaining a sample for use in isolating small molecules.

An adhesive device can be used to achieve such sampling. In preparation for such a sampling study for a dandruff sampling, at a baseline visit, a qualified screening grader will complete adherent scalp flaking score (ASFS) grading for each subject and the highest flaking octant will be identified for tape strip sampling. The highest flaking octant will be sampled at baseline and at various time points during and after treatment with product. Tape strips samples will be collected from each subject at each time point (e.g., baseline and week 3).

The tape strip sampling procedure is repeated additional times, as needed, at the same site placing each D-Squame® tape disc on top of the prior sampled area. The D-Squame® tapes after sample collection are placed into the appropriately labeled wells in a labeled plate.

Following the sampling, an extraction and quantitation procedure is conducted. In an embodiment of the present invention, quantitation of small molecules from extracts of D-Squame® Tape Samples can be conducted via analysis by either LC/MS/MS or GC/MS/MS.
In this embodiment of the present invention, the sample extraction in preparation for Metabolite expression profiling technology was performed.

The D-Squame® tape sample plates are removed from -80°C freezer where they are stored following sample collection, and placed on dry ice. The tape strips are inserted into pre-labeled polypropylene collection tubes, adhesive side facing inward. Extraction buffer containing methanol and water is added to each collection tube and then extracted on ice using sonication for 15 min. If necessary, additional tapes are placed in the collection tube containing the extract solution and the extraction process is repeated as a means of concentrating the sample. Each extract solution is isolated from the tape strip and an aliquot of each sample is placed into a pre-labeled polypropylene collection tubes and frozen at -80°C for metabolite expression profiling analysis. A separate aliquot is specified for soluble protein analysis using a BCA™ Protein Assay Kit. For this analysis, the aliquot consisting of methanol and water is taken to dryness under a gentle stream of nitrogen. The sample is resuspended in an appropriate aqueous buffer (e.g., PBS/SDS (IX PBS + 0.2% SDS) and analyzed for soluble protein.

Following the extraction process, metabolite expression profiling technology is performed as described previously (Lawton et al., 2008). The extracts are analyzed by GC/MS/MS and LC/MS/MS in positive and negative ion mode. Chromatographic separation followed by full scan mass spectra and MS/MS spectra is carried out to record and quantify all detectable ions present in the samples. Metabolites with known chemical structure can be identified by matching the ions' chromatographic retention index and mass spectra fragmentation signatures with reference library entries created from authentic standard metabolites under the identical analytical procedure as the experimental samples. Welch's Two Sample *t*-tests and matched-pair *t*-tests are used to analyze the data. For all analyses, missing values (if any) are imputed with the observed minimum for that particular compound (imputed values are added after block-normalization). The statistical analyses are performed on natural log-transformed data to reduce the effect of any potential outliers in the data. Welch's Two Sample *t*-test comparisons are made between the means of each biochemical from the various groups listed at the top of the tables and are calculated using either or both of the statistical analysis software programs: Array Studio (Omicsoft, Inc) or "R" from the Free Software Foundation, Inc.

### Methodology Extension

Although the exact procedure used is described herein, there are a number of alternate approaches that could be taken for a number of the steps outlined above that are logical extensions. The extraction solvent employed for isolating small molecules from the tape strip can be any appropriate aqueous, organic or organic/aqueous mixture that provides a suitable recovery. LC/MS/MS and GC/MS/MS are generally recognized as the state-of-the-art approaches for the quantitative analysis of small organic molecules in biological matrices due to its high selectivity and sensitivity. However, any analytical technique and or other approach providing the required sensitivity and selectivity could be employed. For example, other methods for assessing small molecule biomarkers have been employed including: capillary electrophoresis, supercritical fluid and other chromatographic techniques and/or combinations thereof with a variety of different detection modes (ex. UV/Vis, fluorescence, ELSD, CAD, MS and MS/MS. Similarly, instrumental approaches without separation techniques have also been employed including nuclear magnetic resonance spectroscopy, mass spectrometry, electrochemical and fluorometric assays. Additionally, ligand binding approaches such competitive and non-competitive enzyme linked immunosorbent assays (ELISAs) and radioimmunoassay (RIA) or other labeling schemes have also been employed. Enzyme-based assays have a long history of use in the analysis of small molecules. Bioassay using either cell-based or tissue-based approaches could have also been used as the means of detection. In an embodiment of the present invention, quantitation of small molecules from hair plucks can be carried out with the same basic extraction and analysis methods as used for tape strip samples.

### Protein Determination of Tape Strip Extracts:

The level of small molecule metabolite on tape strip samples of skin measured using a suitable methodology described above can be standardized using amount of protein found in the tape strip extract. Standardization is done by dividing the small molecule level by the amount of protein in the tape strip extract.

The amount of protein in the tape strip extract or an equivalent matrix that is used to determine the small molecule level on skin can be determined using variety of protein determination methods described in the literature. Examples of such methods include total nitrogen determination, total amino acid determination and protein determination based on any colorimetric, flurometric, and luminometric methods. These methods may or may not involve further sample preparation of the tape strip extract prior to protein determination. A non-limiting example of a specific method for protein determination in the tape strip extract is given below. A comprehensive review of protein determination methods, their applicability and limitations are described in the Thermo Scientific Pierce Protein Assay Technical Handbook that can be downloaded from the following link. www.piercenet.com/Files/1601669_PAssayFINAL_Intl.pdf. Further information related to protein determination can be found at Redinbaugh, M.G. and Turley, R.B. (1986). Adaptation of the bicinchoninic acid protein assay for use with microtiter plates and sucrose gradient fractions. Anal.Biochem. 153, 267-271

Adhesive tapes sampled from human skin will be extracted and analyzed for protein content using the BCA™ Protein Assay Kit. The tape strips sampled from human skin will be extracted with a conventional extraction buffer. Following extraction, aliquots of the tape extracts will be transferred into 96-well polypropylene deep well plates and stored at 2 - 8°C for protein determination.

The BCA™ Protein Assay Kit is based on the reduction of Cu²⁺ to Cu¹⁺ by proteins in an alkaline medium coupled with the sensitive and selective colorimetric detection of Cu⁺¹ by bicinchoninic acid (BCA). The purple-colored reaction product, formed by chelation of two molecules of BCA with one Cu¹⁺ ion, exhibits strong absorbance at a wavelength of 562 nm. The optical density (OD) is measured using a microplate reader. Increasing concentrations of Bovine Serum Albumin (BSA), expressed in micrograms per milliliter (µg/mL), are used to generate a calibration curve in the assay. Appropriate assay QC's prepared from the BSA stock solution will be used to monitor assay performance during sample analysis.

In an alternative embodiment of the present invention, protein determination can be done by direct measurement of protein on an adhesive or an adhesive article such as protein measurement with a SquameScan® 850A (CuDerm Corporation, Dallas, Texas).

### EXAMPLES

### Basic Procedure for Metabolite Expression Profiling Small Molecule Work

Subjects are evaluated by a qualified grader to establish their scalp status. Dandruff subjects are identified by their level of visible flaking as assessed by a qualified grader. Non-dandruff healthy scalp subjects are evaluated at baseline to establish normalized levels of each biomarker. In this study, there are 60 non-dandruff subjects that are evaluated at baseline. There are 119 dandruff sufferers placed on a 1% ZPT containing anti-dandruff shampoo, 115 dandruff sufferers placed on a 1% selenium sulfide containing anti-dandruff shampoo. They are evaluated at baseline, and after 3 weeks of product usage. Subjects undergo a two week washout period with a conventional non-dandruff shampoo without conditioning agents prior to a treatment period with an anti-dandruff shampoo. Dandruff subjects undergo a three week treatment period with an anti-dandruff shampoo (a shampoo composition containing 1% zinc pyrithione or 1% selenium sulfide); tape strip samples are collected from the highest flaking octant as determined at the baseline visit by qualified grader. For dandruff subjects, scalp tape strips are taken at baseline and after a three week product treatment. For non-dandruff subjects, scalp tape strips are taken at baseline only. Tapes are kept at -80°C until extracted. A dandruff-involved site is sampled by parting the hair, applying a D-Squame® tape (CuDerm Corporation), and rubbing the tape, as needed. For the non-dandruff subjects, a flake free site is sampled. The tapes are placed into a pre-labeled sterile 12 well plate for storage.

Samples are extracted with a conventional extraction buffer by sonication on ice. Aliquots of the extracts of D-Squame® Tape samples are then transferred into pre-labeled polypropylene collection tubes and frozen at -80°C for metabolite expression profiling analysis. A separate aliquot is specified for soluble protein analysis using a BCA™ Protein Assay Kit. For this analysis, the aliquot consisting of methanol and water is taken to dryness under a gentle stream of nitrogen. The sample is resuspended in an appropriate aqueous buffer (e.g., PBS/SDS (IX PBS + 0.2% SDS) and analyzed for soluble protein.

Following extraction, the samples are analyzed for metabolite expression profiling by LC/MS/MS (positive and negative ion modes) or GC/MS/MS platforms by Metabolon, Inc. The relative quantitated values for the compounds are then adjusted according to sample volume and standardized to the amount of soluble protein in the extract as determined by the BCA protein assay as outlined above. Analysis is conducted at baseline and week 3 samples from the anti-dandruff shampoo treatment group and for baseline only for the non-dandruff group. Metabolites are detected, and identified based on matched known chemical structures in the Metabolon chemical reference library. Metabolites matching known chemical structures are mapped into their respective general biochemical pathways. Matched pair T-test was used to analyze the differences among the non-dandruff (healthy), dandruff baseline and dandruff treated subjects. Dandruff treated subjects are compared to non-dandruff to determine degree of normalization upon treatment with an anti-dandruff product.

### Results

A total of 255 biochemicals are detected in the tape strips under the metabolite profiling methods and conditions. These biochemicals will provide a rich source of information to further understand skin health. Statistical analysis comparing non-dandruff versus dandruff subjects revealed many biochemicals that can be used to differentiate healthy skin from a skin condition. These biochemicals will be useful as biomarkers to diagnosis the dandruff condition. Many biochemicals are significantly changed between dandruff subjects at baseline and after treatment with an anti-dandruff shampoo. These biochemicals will be useful biomarkers for determining the extent and completeness of a therapeutic improvement of the dandruff condition.

Table 1 contains listed molecules with any statistical significance either between dandruff and non-dandruff samples and before and after three weeks treatment with various anti-dandruff treatments comprising either zinc pyrithione (ZPT) or selenium sulfide. Both treatments are effective anti-dandruff formulas with different anti-dandruff actives. This group of markers demonstrates the difference between dandruff and normal scalp conditions and is useful in evaluating efficacy of anti-dandruff treatment.

Table 2 to Table 7 lists mean values for each biochemical based on their metabolism pathways as well as the p values for comparisons between dandruff and non dandruff, before and after anti-dandruff treatment, and non-dandruff vs. dandruff treatment.

Scalp irritation is a common symptom of dandruff sufferers. It is largely caused by inflammation and host immune responses to microbes. It is known that inflammation can induce cell death which leads to significant structure breakdown for all types of biomolecules including protein, nucleic acids, lipids, and carbohydrates. The listed small molecules including amino acids, amino acid metabolism intermediates (Table 2), dipeptides (Table 3), purine and pyrimidine metabolism intermediates (Table 4), are identified as skin condition biomarkers. The level of these molecules is elevated in the dandruff subjects relative to the non dandruff subjects, and is decreased after treatment with anti-dandruff shampoo indicating an improvement of the dandruff condition. This is consistent with self assessed data from the same clinical subjects who reported less scalp irritation after treatment with an anti-dandruff shampoo. These metabolites may be used as objective measures of skin irritation.

It is believed that some biochemicals may increase inflammation by generating oxidative stress. For example, metabolism of purine can serve as an indication of an increased inflammatory response (i.e. Table 4). Specifically, the conversion of hypoxanthine to xanthine and the subsequent conversion of xanthine to uric acid require oxygen to activate xanthine oxidase, a major source of H₂O₂ generation. In the context of human skin (e.g. dandruff), increased oxidative stress would result in higher levels of purine degradation, reflected in increased levels of hypoxanthine and xanthine. The significantly increased levels of adenosine, guanine, guanosine, inosine, hypoxanthine, and xanthine in tape strips obtained from dandruff sufferer's scalps suggested increased oxidative stress and thus these metabolites are useful biomarkers for assessing oxidative stress. As an example, the baseline level of xanthine in dandruff suffers have a 13.72 fold increase (p<0.05) of xanthine as compared to a non-dandruff group wherein the level of xanthine in dandruff sufferers after three weeks of anti-dandruff treatment with ZPT was significantly decreased as compared to baseline (0.16 fold change (i.e., an 84% reduction), p<0.05), and was not statistically different as compared to non dandruff group, suggesting the dandruff condition has been improved. Another anti dandruff treatment with selenium sulfide has similar fold change patterns for xanthine as treatment with ZPT. For the selenium sulfide treatment group, the baseline level of xanthine in dandruff suffers have a 15.96 fold increase (p<0.05) of xanthine as compared to non-dandruff group wherein the level of xanthine in dandruff sufferers after three weeks of anti-dandruff treatment with selenium sulfide is significantly decreased as compared to baseline (0.15 fold change (i.e., 85% decrease), p<0.05). When comparing the levels of xanthine in dandruff treated subjects to the healthy subjects the fold changes were reduced from 13.72 before treatment with ZPT to 2.19 after treatment, and 15.96 before treatment with selenium sulfide to 2.37 after treatment. This represents a substantial normalization post treatment.

### Lipid metabolism intermediates

Lipids have multiple biological functions and this is also reflected in their metabolism pattern. A number of lipids display a significant difference between dandruff and non-dandruff subjects and for dandruff subjects treated with an anti-dandruff shampoo (Table 5). The overall trend observed is higher levels of fatty acids in the dandruff subjects versus non dandruff and a decrease in fatty acid levels in the dandruff subjects after treatment with an anti-dandruff shampoo. For example, linoleate was significantly higher in dandruff versus non-dandruff (ZPT and selenium sulfide baseline dandruff groups demonstrate 2.45 and 1.6 fold increase versus non-dandruff group, respectively). After treatment with anti-dandruff shampoo the levels are decreased in dandruff sufferers resulting in fold changes of 0.18 for ZPT shampoo (i.e., 82% decrease) and 0.22 for selenium shampoo (i.e., 78% decrease) when compared to baseline dandruff levels; both with statistical significance. This observation is somewhat counterintuitive as structural lipids are typically decreased in dandruff subjects as a result of an impaired barrier. A reasonable explanation for this observance is that by sampling the scalp surface, what is primarily sampled is a result of triglycerides in the sebum that are being converted into fatty acids for use as a food source by the scalp microflora (Malassezia). An additional explanation is that an excess of fatty acids are necessary to repair cell damage including the recovery of the skin barrier integrity. Thus, decreased levels of fatty acids levels indicate the improvement of the scalp health and function with an effective anti-dandruff shampoo.

Several lipid compounds are important for the initiation and propagation of inflammatory signaling in cells and tissues. These lipid signaling inflammatory mediators are liberated from the plasma membrane phospholipids by phospholipase A₂ enzymes. Arachidonic acid release can be initiated by tissue damage which increases during the early stages in the inflammatory process. Dandruff subjects treated with 1% ZPT or 1% selenium sulfide shampoo have significantly lower levels of arachidonic acid when compared to dandruff baseline. Fold change of 0.56 is observed for ZPT (i.e., 44% decrease, p-value <0.05) and 0.30 fold change for selenium sulfide (i.e., 70% decrease, p-value <0.05) which is consistent with an improvement in the inflammatory phenotype of the dandruff condition.

Further evidence of the importance of lipids in scalp health is the detection of the oxidized form of linoleic acid, fatty acid 13- and 9-hydroxyoctadecadienoic acid (13-HODE, 9-HODE). The levels of HODE are relatively high in dandruff vs. non dandruff sufferers (fold change: 1.93 for ZPT shampoo, 1.41 for selenium shampoo). After three weeks anti-dandruff shampoo treatment, the level of 13-HODE + 9-HODE changes significantly in comparison to the matched baseline samples. Fold change of 13-HODE + 9-HODE for ZPT treatment is 0.26 (i.e., 74% decrease, 0.05 ≤ p ≤ 0.10), and for selenium sulfide treatment is 0.34 (i.e., 66% decrease, p ≤ 0.05). It is believed that increased lipid peroxidation is a signature of elevated oxidative stress impacting the plasma membrane. The formation of monohydroxy fatty acids, including 13-HODE, 9-HODE, resulting from the oxidation of linoleic acid is indicative of lipid peroxidation and oxidative stress. Treatment of dandruff with anti-dandruff shampoo resulted in a significant decrease in 13-HODE/9-HODE compared to the dandruff samples. This is consistent with the observation of higher levels of purine degradation and taken together suggests that the dandruff phenotype displays inflammatory and oxidative stress components and that treatment with an anti-dandruff shampoo provides a therapeutic improvement.

An additional example of an important lipid for scalp health that exhibits a different type of metabolism pattern is Palmitoylethanolamide (PEA). Unlike 13-HODE/9-HODE, (PEA) displayed significantly higher levels in dandruff treated with an anti-dandruff shampoo compared to dandruff sufferers. The PEA level is about the same between dandruff and non-dandruff groups (fold change for ZPT: 1.44; fold change for selenium sulfide: 0.79; both without statistical significance). However, after three weeks of ZPT treatment, PEA level goes up to 3.08 fold for ZPT shampoo, and 6.69 fold increase for selenium sulfide shampoo, p≤0.05. Also, both anti-dandruff treatments significantly increased the fold change of PEA level between three week treatment and non-dandruff groups (4.43, and 5.26 fold for ZPT and selenium sulfide, respectively, p≤0.05). PEA, as an endogenous fatty acid amide, has been demonstrated to exert a great variety of biological functions related to chronic pain, itch and inflammation. An effective anti-dandruff shampoo treatment may enhance PEA for its anti-inflammatory and anti-pruritic functions as part of therapeutic mechanisms. This is consistent with self assessed data from the same clinical subjects who report less scalp irritation and less scalp itch after treatment with an anti-dandruff shampoo (Table 8). The significant fold change data between three week treatments and non-dandruff group are observed (Fold change 4.43 for ZPT, and 5.26 for Selenium sulfide, both p≤0.05.) It is hypothesized that at week three treatment time point, dandruff scalp is still exhibiting some inflammation and that an extra amount of PEA is needed.

Table 6 listed metabolites involved in carbohydrates and their metabolites, intermediates in energy cycles, and enzyme co-factors. Shown are biomolecules displaying significant difference either between dandruff and non-dandruff samples or before and after three weeks treatment with various anti-dandruff treatments comprising either zinc pyrithione (ZPT) or selenium sulfide, both treatments that are effective anti-dandruff formulas with different anti-dandruff actives. The results demonstrate the difference between dandruff and normal scalp conditions and between dandruff baseline and dandruff treated conditions and this is useful in evaluating the efficacy of anti-dandruff treatments.

Metabonomics technology includes two major technical aspects: metabolites separation and metabolites identification. Although the identities of some of the metabolites and small molecule compounds are not known at this time, such identities are not necessary for the identification of the metabolites or small molecule compounds in biological samples from subjects, as the "unnamed" compounds have been sufficiently characterized by analytical techniques to allow such identification. The analytical characterization of all such "unnamed" compounds is listed in Table 7. Such "unnamed" metabolites and small molecule compounds are designated herein using the nomenclature "X" followed by a specific compound number. Table 7 lists 45 metabolites having significant statistical difference either between dandruff and non-dandruff samples or before and after three weeks treatment with various anti-dandruff treatments comprising either zinc pyrithione (ZPT) or selenium sulfide, or both treatments. These compounds can also be used as markers for distinguishing dandruff from non-dandruff and/or efficacy indicators for anti-dandruff shampoo treatments.

In order to assess the signs and symptoms associated with dandruff a questionnaire was used. This information will provide an independent measure of the status of scalp health. For the self assessment questions, dandruff subjects answered the four questions listed below pertaining to the severity of their dandruff signs and symptoms at baseline and after 3 weeks of product use. The scale the subjects used was represented from 0 to 4 according to the description below.

### Scales:

### 1. Self Assessment Scale (0-4)

0 = none, 1= mild, 2= moderate, 3 = severe, 4= very severe

### 2. Expert Grading Scale (0-80)

Dandruff ≥24
Non-Dandruff ≤8

### Self Assessment Questions:

How would you rate the severity of your itch today?
How would you rate the severity of your irritation today?
How would you rate the severity of your dryness today?
How would you rate the severity of your flaking today?

### Relationship of Small Molecule Biomarkers to Self Assessment/Clinical Symptoms

Clinical subjects reported a decrease in their signs and symptoms of dandruff after a 3 week treatment period with anti-dandruff shampoo (Table 8). An expert grader also reports a substantial decrease in flaking score after treatment with anti-dandruff shampoo. These results are consistent with an overall improvement in scalp health. The biochemical molecules observed for these same subjects are also consistent with an overall improvement in scalp health. For example, irritation is highly associated with inflammation and host immune defense reactions. Many molecules revealed by this invention such as high level of free amino acids, dipeptides, nucleic acids as well as lipids involved in inflammatory signaling in dandruff subjects strongly support the link between the irritation experienced by dandruff sufferers and these molecular indicators of inflammation. This direct correlation has provided an objective measurement for the perception of irritation. Subjective measures are commonly used to assess skin conditions and typically involve either expert graders and/or self-assessment. While these types of measures are useful, they are inherently biased by the evaluator. Objective measures do not have an opportunity for bias and therefore represent more rigorous scientific data.

Overall, these small molecule biomarkers can serve as objective measures to enable a more detailed pathophysiological description of the dandruff condition as well as serve as relevant measures that are indicative of the extent and completeness of therapeutic resolution of dandruff.

In particular embodiments, such performance assessments can be advantageous for comparing a subject's skin health status before and after treatment with a composition. For example, some anti-dandruff compositions may promote a faster speed of relief that will be experienced by the subject if the subject switches to and maintains a certain anti-dandruff composition or regimen. An assessment that allows objective measurement of particular skin health benefits of the subject could allow for the comparing of a subject's status before the treatment with a particular anti-dandruff composition or regimen and after the subject switches to the particular anti-dandruff composition or regimen. Additionally, such an assessment could be used as a supporting credentialing tool that supports particular skin health benefit claims that the particular treatment or regimen is promoting. For example, if a particular composition or regimen is promoting that it will decrease the symptoms of itch, an assessment as disclosed herein can be used to support the specific health benefit claim to show that the subject's discomfort from itch has decreased. Non-limiting elements of skin health that could be benefited by anti-dandruff compositions and regimens include: itch, flaking, irritation, skin barrier integrity, dryness, oxidative stress and oxidative damage, self-defense, natural protection.

The results of many analyses may also be used as marketing or advertising information to promote the effectiveness of particular products, combinations of products, and techniques. Examples of advertising claims that could be placed on product packaging that might be substantiated by the present invention include, but are not limited to, establishment claims (e.g., "clinically proven" or "tests show"), before and after claims (e.g., "50% less dandruff after use"), monadic claims, comparative claims, factor-claims (e.g., "3X reduction in dandruff"), and prevention and treatment claims. For example, product packages may refer to an analysis and demonstrate objectively-proven effectiveness or comparisons of the product. Also, analysis data may be used in clinical information related to different regimen that may or may not be used in combination with different products or groups of products.

A further embodiment includes wherein there is a change in standardized biomarker following application with a zinc pyrithione shampoo when compared to a baseline level of biomarker prior to the application. In another embodiment, there is a change in standardized molecule biomarker following application with a selenium sulfide shampoo when compared to a baseline level of biomarker prior to the application. In a further embodiment, there may be a measure of biomarker that establishes an improvement of at least 5% in skin health compared to a normal population following treatment. In a further embodiment, there may be an improvement of at least 10%, at least 20%, at least 30%, at least 40%, at least 50%, at least 60%, at least 70%, at least 80%, at least 90% and further, 100% improvement in skin health compared to a normal population following treatment. In another embodiment, there may be at least a 5% difference between dandruff and non-dandruff (no treatment). Further, there may be at least 10% difference between dandruff and non-dandruff, at least 20%, at least 30%, at least 40%, at least 50%, at least 60%, at least 70%, at least 80%, at least 90%, between dandruff and non-dandruff and further, 100% or greater difference between dandruff and non-dandruff.

The dimensions and values disclosed herein are not to be understood as being strictly limited to the exact numerical values recited. Instead, unless otherwise specified, each such dimension is intended to mean both the recited value and a functionally equivalent range surrounding that value. For example, a dimension disclosed as "40 mm" is intended to mean "about 40 mm."

**Table 1a. Fold Change of Small Molecules**

| | **Fold Change** | | | | | |
|---|---|---|---|---|---|---|
| **Biochemical Name** | **Wk3 ZPT vs Dandruff Baseline** | **Wk3 Sel vs Dandruff Baseline** | **ZPT Baseline vs Non-Dan Baseline** | **Sel Baseline vs Non-Dan Baseline** | **Wk3 ZPT vs Non-Dan Baseline** | **Wk3 Sel vs Non-Dan Baseline** |
| Glycine | **0.6*** | 0.62 | **2.16**** | **2.28**** | 1.3 | **1.41****** |
| Serine | **0.64*** | 0.68 | **1.94**** | **2.06**** | 1.23 | **1.4****** |
| N-acetylserine | **0.52*** | 0.54 | **2.54**** | **2.79**** | 1.32 | **1.51****** |
| Threonine | **0.68*** | **0.72***** | **1.64****** | **1.75**** | 1.12 | **1.27**** |
| Aspartate | **0.71*** | **0.58***** | **1.84**** | **2.07**** | 1.31 | 1.19 |
| Asparagine | **0.48** | **0.45*** | **2.83**** | **3.24**** | 1.36 | **1.47****** |
| beta-alanine | 1.69 | **2.11****** | **0.47*** | **0.59*** | 0.79 | 1.25 |
| Alanine | **0.64*** | 0.81 | **1.78**** | **1.84**** | 1.15 | **1.5**** |
| N-acetylalanine | 0.85 | **1.28**** | 1.28 | 1.46 | 1.09 | **1.87****** |
| Glutamate | 0.7 | 0.76 | **1.55**** | **1.91**** | 1.09 | **1.45****** |
| Glutamine | 0.82 | 1.42 | 1.39 | 1.53 | 1.14 | **2.16**** |
| Gamma-aminobutyrate (GABA) | **0.09*** | **0.08*** | 1.72 | **2.51**** | **0.15*** | **0.21***** |
| Histidine | 0.8 | 0.83 | 1.37 | 1.54 | 1.1 | **1.29**** |
| trans-urocanate | 0.87 | **0.74*** | **1.63**** | **1.64**** | 1.41 | 1.21 |
| Histamine | **0.07*** | **0.06*** | **14.45**** | **14.31**** | 1.02 | 0.88 |
| imidazole propionate | **0.72*** | 0.47 | 0.76 | 1.12 | **0.55***** | **0.53***** |
| cis-urocanate | 1.19 | 1.22 | **0.76*** | 0.81 | 0.91 | 0.99 |
| Lysine | **0.43***** | **0.51***** | **3.45**** | **4.52**** | **1.48**** | **2.29**** |
| phenyllactate (PLA) | 0.64 | **0.33*** | 1.01 | **2.01****** | 0.65 | 0.66 |
| Phenylalanine | **0.63*** | **0.65***** | **2.12**** | **2.34**** | 1.33 | **1.53**** |
| Tyrosine | **0.6*** | **0.64***** | **2.17**** | **2.31**** | 1.3 | **1.47**** |
| 3-(4-hydroxyphenyl)lactate | **0.7***** | **0.44*** | 1.15 | **1.91**** | 0.8 | 0.84 |
| Tryptophan | **0.62*** | 0.65 | **2.07**** | **2.31**** | 1.28 | **1.5**** |
| Isoleucine | **0.68*** | 0.75 | **1.86****** | **1.98**** | 1.27 | **1.49**** |
| Leucine | **0.6*** | **0.64***** | **2.26**** | **2.44**** | **1.35**** | **1.56**** |
| Valine | **0.73***** | 0.76 | **1.57****** | **1.73**** | 1.15 | **1.31**** |
| Cysteine | **0.73*** | 0.9 | **1.38**** | 1.56 | 1 | **1.41**** |
| methionine sulfoxide | **0.42*** | **0.46*** | 2.02 | **1.95**** | 0.86 | 0.9 |
| Methionine | **0.71***** | 0.77 | **1.63****** | **1.82**** | 1.16 | **1.4****** |
| N-acetylmethionine | **0.37*** | **0.28*** | **3.91**** | **4.4**** | 1.46 | 1.25 |
| Arginine | **0.58***** | 0.83 | **1.52****** | **1.59**** | 0.89 | 1.32 |
| Ornithine | **1.76**** | 0.87 | **1.43****** | **1.67****** | **2.5**** | **1.46****** |
| Urea | 4.13 | **2.81****** | 0.91 | 1.29 | 3.75 | **3.62**** |
| Proline | **0.65*** | **0.6*** | **1.81**** | **1.98**** | 1.18 | 1.19 |
| Citrulline | **0.7*** | **0.67***** | 1.4 | 1.4 | 0.98 | 0.94 |
| N-acetylornithine | 1.2 | **0.58***** | **0.6*** | 0.95 | 0.72 | **0.55***** |
| Putrescine | **0.12***** | **0.13*** | 3.11 | **5.55**** | 0.38 | 0.71 |

| Table 1b | **Fold Change** | | | | | |
|---|---|---|---|---|---|---|
| **Biochemical Name** | **Wk3 ZPT vs Dandruff Baseline** | **Wk3 Sel vs Dandruff Baseline** | **ZPT Baseline vs Non-Dan Baseline** | **Sel Baseline vs Non-Dan Baseline** | **Wk3 ZPT vs Non-Dan Baseline** | **Wk3 Sel vs Non-Dan Baseline** |
| Glycylglycine | **0.69***** | 0.73 | **1.77**** | **1.9**** | 1.22 | 1.39 |
| Glycylproline | **0.63*** | 0.78 | **1.62**** | **1.83**** | 1.02 | **1.42**** |
| Glycylisoleucine | 1 | 0.89 | **1.56****** | **1.77**** | **1.56**** | **1.58**** |
| Glycylleucine | 0.88 | 0.92 | **1.79**** | **2.08**** | **1.56**** | **1.92**** |
| alanylphenylalanine | **0.76***** | 0.8 | **1.65****** | **2.69**** | 1.26 | **2.14**** |
| Arginylproline | 0.54 | **0.61*** | 1.62 | **2.14**** | 0.88 | 1.3 |
| Leucylleucine | 0.7 | 1 | 1.18 | 1.49 | **0.83***** | **1.48**** |
| pyroglutamylvaline | 0.77 | 0.72 | **1.46**** | **1.57**** | 1.12 | 1.14 |
| Valylserine | 0.65 | **0.61***** | **1.86**** | **2.32**** | 1.21 | **1.4**** |
| isoleucylglycine | 0.85 | 0.77 | **1.54****** | **1.76**** | 1.31 | **1.36****** |
| Isoleucylserine | 0.63 | 0.76 | **1.88****** | **1.95**** | 1.19 | **1.48**** |
| Leucylglutamate | 1.13 | **0.81*** | 0.82 | 1.26 | 0.93 | 1.02 |
| Leucylglycine | 0.81 | 0.81 | **2.03**** | **2.05**** | **1.65**** | **1.66**** |
| Leucylserine | 0.8 | 0.98 | **1.94****** | 2.11 | **1.56**** | **2.06**** |
| Leucylvaline | **1.67**** | **2.17****** | **0.62*** | **0.62*** | 1.03 | 1.35 |
| Threonylleucine | 0.78 | 0.73 | 1.12 | **1.53**** | 0.87 | 1.11 |
| Valylisoleucine | **0.87*** | 0.74 | 1.23 | **1.45****** | 1.07 | 1.07 |
| Valylhistidine | 0.72 | 0.66 | **1.71****** | **2.08****** | 1.24 | **1.38**** |
| Gamma-glutamylvaline | 0.86 | 0.73 | **1.53**** | **1.76**** | 1.32 | 1.28 |
| Gamma-glutamylleucine | 0.76 | **0.57*** | **2.09**** | **2.51**** | **1.58**** | 1.43 |
| Gamma-glutamylisoleucine* | 0.9 | **0.73*** | **1.84**** | **2.21**** | **1.64**** | **1.61**** |
| Gamma-glutamylglutamate | **1.89**** | 0.95 | 1.1 | 1.17 | **2.09**** | 1.11 |
| Gamma-glutamylphenylalanine | 0.93 | **0.72*** | 1.46 | **1.82****** | 1.35 | 1.32 |
| Gamma-glutamyltyrosine | 0.9 | **0.63***** | **1.75**** | **2.14**** | **1.57****** | **1.34****** |
| Gamma-glutamylthreonine* | 0.9 | **0.58*** | 1.28 | **1.5**** | 1.15 | 0.87 |
| Mannitol | **0.25***** | **0.04*** | 1.04 | 1.73 | 0.26 | **0.08*** |
| Trehalose | 1.16 | **0.36*** | 0.45 | 0.88 | 0.52 | **0.32***** |
| Glucose | 1.02 | **0.72*** | **0.31***** | 0.37 | **0.32***** | **0.26***** |
| Pyruvate | **5.1****** | **4.38**** | 0.54 | 0.81 | 2.77 | **3.53**** |
| Lactate | **4**** | **5.39**** | 0.86 | 0.88 | 3.43 | **4.75**** |
| Arabitol | **0.61*** | 0.73 | 1.06 | 1.46 | 0.65 | 1.07 |
| Ribitol | **0.13*** | **0.09*** | **2.2****** | **4.21**** | **0.29*** | **0.36*** |
| Fumarate | 0.53 | **0.37***** | 1.27 | **2.04**** | 0.68 | 0.76 |
| Malate | **0.2*** | **0.19*** | **2.27**** | **2.43**** | **0.44*** | **0.46*** |
| Phosphate | 0.62 | 0.7 | **1.68****** | **1.78**** | 1.03 | 1.25 |
| linoleate (18:2n6) | 0.18 | **0.22*** | 2.45 | **1.6**** | **0.43*** | **0.35*** |

| Table 1c | **Fold Change** | | | | | |
|---|---|---|---|---|---|---|
| **Biochemical Name** | **Wk3 ZPT vs Dandruff Baseline** | **Wk3 Sel vs Dandruff Baseline** | **ZPT Baseline vs Non-Dan Baseline** | **Sel Baseline vs Non-Dan Baseline** | **Wk3 ZPT vs Non-Dan Baseline** | **Wk3 Sel vs Non-Dan Baseline** |
| linolenate [alpha or gamma; (18:3n3 or 6)] | 0.85 | **0.67***** | 0.83 | 0.79 | **0.7***** | **0.53*** |
| myristate (14:0) | 0.87 | **0.6*** | **0.69*** | 0.74 | **0.6*** | **0.45*** |
| Myristoleate (14:1 n5) | **0.8***** | **0.53***** | 0.75 | 0.86 | **0.6*** | **0.45*** |
| pentadecanoate (15:0) | **0.4*** | **0.19*** | 0.92 | 1.03 | **0.37*** | **0.19*** |
| palmitate (16:0) | 0.95 | **0.6*** | **0.61***** | **0.69***** | **0.58*** | **0.42*** |
| Palmitoleate (16:1 n7) | **0.83*** | **0.55*** | **0.7***** | 0.78 | **0.58*** | **0.43*** |
| margarate (17:0) | **0.6***** | **0.31*** | 0.76 | 0.81 | **0.45*** | **0.25*** |
| 10-heptadecenoate (17:1 n7) | **0.67***** | **0.33*** | 0.78 | 0.88 | **0.52***** | **0.29*** |
| stearate (18:0) | 1.03 | 0.77 | **0.66***** | **0.67***** | 0.67 | **0.52*** |
| oleate (18:1 n9) | **0.51***** | **0.33*** | 0.96 | 0.9 | **0.49*** | **0.3*** |
| nonadecanoate (19:0) | **0.47*** | **0.29*** | 0.84 | 0.91 | **0.39*** | **0.26*** |
| 10-nonadecenoate (19:1 n9) | **0.31*** | **0.14*** | 1.23 | 1.26 | **0.38*** | **0.17*** |
| arachidate (20:0) | **0.55*** | **0.42*** | 1.02 | 1.07 | **0.56***** | **0.45***** |
| Eicosenoate (20:1 n9 or 11) | **0.27*** | **0.16*** | 1.27 | 1.25 | **0.34***** | **0.2*** |
| dihomo-linoleate (20:2n6) | **0.3*** | **0.15*** | 1.07 | 1.16 | **0.32*** | **0.17*** |
| arachidonate (20:4n6) | **0.56*** | **0.3*** | 0.87 | 1.04 | 0.49 | **0.31*** |
| behenate (22:0) | 0.47 | 0.84 | 0.72 | 0.68 | **0.34*** | **0.57***** |
| docosadienoate (22:2n6) | **0.24*** | **0.12*** | 1.14 | 1.15 | **0.28*** | **0.14*** |
| docosatrienoate (22:3n3) | **0.26*** | **0.13*** | 1.24 | 1.15 | 0.32 | **0.15*** |
| 2-hydroxypalmitate | 1.04 | **0.57*** | 0.57 | 0.86 | **0.6*** | **0.49*** |
| 13-HODE + 9-HODE | **0.26***** | **0.34*** | 1.93 | 1.41 | **0.5***** | **0.48*** |
| Oleamide | **4.24**** | **1.64** | **0.07***** | 0.14 | **0.28** | 0.23 |
| 11-methyllauric acid | **0.3*** | **0.26***** | 1.63 | 1.76 | **0.48***** | **0.45*** |
| 12-methyltridecanoic acid | **0.29*** | **0.17*** | 1.19 | 1.31 | **0.34*** | **0.22*** |
| 13-methylmyristic acid | **0.37*** | **0.21*** | 0.85 | 1.14 | **0.31*** | **0.24*** |
| 15-methylpalmitate | **0.4*** | **0.31*** | 0.86 | 0.89 | **0.35*** | **0.27*** |
| isopalmitic acid | **0.41*** | **0.23*** | 1 | 1.06 | **0.41*** | **0.24*** |
| 17-methylstearate | **0.36*** | **0.21*** | 0.96 | 1.01 | **0.35*** | **0.22*** |
| 19-methylarachidic acid | 0.99 | **0.46***** | **0.61*** | 1.05 | 0.6 | **0.48*** |
| 2-methylpalmitate | **0.17*** | **0.1*** | 1.14 | **1.39**** | **0.2*** | **0.14*** |
| Palmitoyl ethanolamide | 3.08 | **6.69**** | 1.44 | 0.79 | **4.43**** | **5.26**** |
| Stearoyl ethanolamide | 2.94 | **5.03**** | 1.32 | 0.88 | **3.88**** | **4.41**** |
| 1-hexadecanol | 0.79 | **0.3*** | 0.81 | 0.88 | 0.64 | **0.27*** |
| Ethanolamine | 1.08 | **1.63****** | **2.36**** | **2.75**** | **2.53****** | **4.49**** |
| Diethanolamine | 0.91 | **1.83**** | 0.66 | 0.42 | 0.6 | 0.76 |

| Table 1d | **Fold Change** | | | | | |
|---|---|---|---|---|---|---|
| **Biochemical Name** | **Wk3 ZPT vs Dandruff Baseline** | **Wk3 Sel vs Dandruff Baseline** | **ZPT Baseline vs Non-Dan Baseline** | **Sel Baseline vs Non-Dan Baseline** | **Wk3 ZPT vs Non-Dan Baseline** | **Wk3 Sel vs Non-Dan Baseline** |
| Glycerol | **0.4*** | **0.17*** | **2.37**** | **3.27**** | 0.95 | **0.56***** |
| 1-myristoylglycerol (1-monomyristin) | 0.94 | **0.43*** | 1 | 1.39 | 0.94 | 0.59 |
| 1-pentadecanoylglycerol (1-monopentadecanoin) | 0.95 | **0.44*** | 1.04 | 1.22 | 0.99 | 0.54 |
| 2-palmitoylglycerol (2-monopalmitin) | **1.57**** | **1.36****** | 0.66 | 0.88 | 1.03 | 1.2 |
| 1-stearoylglycerol (1-monostearin) | **2.59**** | **2.25**** | **0.4*** | **0.52*** | 1.03 | 1.17 |
| 2-stearoylglycerol (2-monostearin) | **1.69**** | **1.94**** | 0.63 | **0.68*** | 1.07 | 1.32 |
| 1-oleoylglycerol(1-monoolein) | **0.63***** | **0.47*** | 1.11 | 1.1 | 0.7 | 0.51 |
| Sphinqosine | 1.82 | **2.45****** | 0.99 | 0.93 | 1.8 | **2.28**** |
| Cholesterol | 1.36 | 1.64 | 0.93 | 0.86 | 1.27 | **1.42**** |
| Xanthine | **0.16*** | **0.15*** | **13.72**** | **15.96**** | 2.19 | **2.37****** |
| hypoxanthine | **0.21*** | **0.22*** | **7.85**** | **8.84**** | **1.67**** | **1.95**** |
| Inosine | **0.35*** | **0.34*** | **2.83**** | **3.07**** | 1 | 1.04 |
| Adenosine | **0.43*** | **0.42*** | 1.65 | **1.96****** | 0.7 | 0.82 |
| N1 -methyladenosine | **0.38*** | **0.32*** | **2.02** | **2.44**** | 0.77 | 0.77 |
| Guanine | **0.19*** | **0.27*** | **3.5**** | **3.97** | 0.66 | 1.07 |
| Guanosine | **0.42*** | **0.41***** | **2.61**** | **2.97**** | 1.09 | 1.21 |
| Allantoin | **0.69***** | **0.46*** | 1.33 | **3.53****** | 0.91 | 1.61 |
| Cytidine | **0.21*** | **0.22*** | **6.5**** | **7.19**** | 1.39 | 1.62 |
| Uridine | **0.41*** | **0.43*** | **2.14**** | **2.23**** | **0.88***** | 0.95 |
| N1 -Methyl-2-pyridone-5-carboxamide | 1.57 | 0.89 | **0.49*** | 0.6 | 0.76 | **0.54*** |
| pantothenate | **0.29*** | **0.24*** | **1.98****** | **2.42**** | **0.58*** | **0.58*** |
| Benzoate | **2.02****** | **7.26****** | 1.07 | **0.59*** | **2.16****** | **4.26****** |
| 2-amino-2-methyl-1-propanol | **2.75**** | 1.92 | **0.45*** | 0.6 | **1.24****** | 1.15 |
| | | | | | | |
| | | | | | | |

| **Name** | | | | | | |
|---|---|---|---|---|---|---|
| X - 11297 | 2.47 | **3.11**** | **0.56***** | **0.5*** | 1.38 | 1.55 |
| X - 11509 | **1.64**** | 1.6 | **0.51*** | **0.51*** | 0.83 | 0.81 |
| X - 11533 | **3.18****** | **2.67**** | **0.52*** | **0.59*** | 1.67 | **1.56**** |
| X - 11543 | **1.62**** | 1.54 | **0.53***** | **0.57***** | 0.86 | 0.88 |
| X - 12565 | **0.78*** | **0.77***** | 1.24 | 1.41 | 0.97 | 1.09 |
| X - 12776 | **2.73****** | **3.29****** | 0.49 | 0.69 | 1.33 | 2.27 |
| X - 13005 | **2.93**** | 1.12 | 0.35 | 0.53 | 1.02 | 0.59 |
| X - 13081 | 2.57 | **4.74**** | 0.68 | **0.44*** | 1.74 | **2.1**** |

| Table 1e | **Fold Change** | | | | | |
|---|---|---|---|---|---|---|
| | | | | | | |
| **Name** | **Wk3 ZPT vs Dandruff Baseline** | **Wk3 Sel vs Dandruff Baseline** | **ZPT Baseline vs Non-Dan Baseline** | **Sel Baseline vs Non-Dan Baseline** | **Wk3 ZPT vs Non-Dan Baseline** | **Wk3 Sel vs Non-Dan Baseline** |
| X - 13230 | **2.36****** | 1.69 | **0.36*** | **0.48***** | 0.85 | 0.82 |
| X - 13372 | 1.34 | 1.32 | 0.57 | **0.58***** | 0.77 | **0.76*** |
| X - 13504 | 0.86 | 1.07 | **1.41**** | 1.42 | 1.22 | **1.51****** |
| X - 13529 | 0.93 | 0.9 | **1.32****** | **1.54**** | 1.23 | **1.38****** |
| X - 13582 | 0.81 | **0.7*** | 1.13 | 1.03 | 0.91 | 0.72 |
| X - 13668 | 0.42 | **0.47*** | 1.45 | 1.21 | **0.61** | **0.57*** |
| X - 13737 | **0.35*** | **0.45*** | 1.79 | 1.42 | 0.62 | 0.64 |
| X - 13828 | **1.85****** | **2.1****** | 0.48 | **0.46*** | 0.88 | 0.96 |
| X - 14097 | **0.69*** | 0.72 | **1.98****** | **1.89**** | 1.37 | **1.36**** |
| X - 14196 | **0.77*** | 0.8 | **1.47**** | **1.54****** | 1.13 | 1.23 |
| X - 14198 | 0.71 | **0.79*** | 1.77 | **2.1**** | 1.26 | **1.65****** |
| X - 14302 | **0.75*** | 0.72 | **1.58**** | **1.68**** | 1.18 | 1.21 |
| X - 14314 | **0.72*** | 0.69 | **1.83**** | **1.96**** | **1.31****** | **1.36****** |
| X - 14427 | 0.85 | 0.7 | 1.33 | **1.58**** | 1.13 | 1.11 |
| X - 14445 | 0.74 | 0.76 | **1.67****** | **1.75**** | 1.23 | 1.33 |
| X - 14904 | 0.05 | **0.3*** | 13.19 | **2.82**** | 0.7 | 0.84 |
| X - 15657 | 1.2 | 1.45 | **0.59***** | **0.53*** | **0.7*** | 0.76 |
| X - 15664 | 1.34 | 1.89 | **0.42*** | **0.41*** | 0.57 | 0.78 |
| X - 15782 | **1.54**** | 1.5 | **0.53*** | **0.56*** | 0.81 | 0.84 |
| X - 15808 | 1.39 | **2.29****** | **0.4***** | **0.42*** | **0.56*** | 0.97 |
| X - 16212 | 1.32 | 1.11 | 0.52 | **0.58*** | **0.69***** | **0.64*** |
| X - 16468 | **0.78*** | 0.75 | 1.21 | 1.43 | 0.95 | 1.08 |
| X - 16626 | 1.21 | 1.66 | 0.72 | **0.5*** | 0.87 | 0.83 |
| X - 17375 | **1.5**** | 1.46 | **0.52***** | **0.56*** | 0.78 | 0.81 |
| X - 17553 | 1.6 | **0.62***** | **0.33*** | **0.42***** | 0.52 | **0.27*** |
| X - 17971 | **0.13*** | **0.21*** | **4.29**** | **2.73****** | 0.54 | 0.57 |
| X - 18113 | 1.2 | **1.23**** | 0.96 | 0.6 | 1.15 | 0.73 |
| X - 18309 | 0.95 | 1.67 | 0.95 | **0.48*** | 0.9 | 0.8 |
| X - 18341 | **0.79***** | 0.76 | **1.78**** | **1.94****** | 1.4 | **1.47**** |

| | | | | | | |
|---|---|---|---|---|---|---|
| * p ≤ 0.05, ratio < 1 ** 0.05 ≤ p ≤ 0.10, ratio ≥ 1 *** p ≤ 0.05, ratio < 1 **** 0.05 ≤ p ≤ 0.10, ratio ≥ 1 | | | | | | |

**Table 2a. Amino Acids and their Metabolites**

| | **Mean Values** | | | | | **Statistical P Values** | | | | | |
|---|---|---|---|---|---|---|---|---|---|---|---|
| | NON-Dan BASELINE | DANDRUFF BASELINE ZPT | DANDRUFF BASELINE Sel | WEEK 3 TRT ZPT | WEEK 3 TRT Sel | | | | | | |
| **Biochemical Name** | | | | | | Dan Baseline ZPT vs Non Dan Baseline | Dan Baseline Sel vs Non Dan Baseline | Dan ZPT W3 vs Dan ZPT Baseline | Dan Sel W3 vs Dan Sel Baseline | Dan ZPT W3 vs Non Dan Baseline | Dan Sel W3 vs Non Dan Baseline |
| Glycine | 0.6143 | 1.3263 | 1.4025 | 0.7968 | 0.8645 | 0.005 | 0.0068 | 0.0234 | 0.1057 | 0.2093 | 0.066 |
| Serine | 0.6754 | 1.308 | 1.3885 | 0.8335 | 0.9489 | 0.0092 | 0.0066 | 0.0089 | 0.1177 | 0.168 | 0.0776 |
| N-acetylserine | 0.6204 | 1.5754 | 1.7327 | 0.8203 | 0.9398 | 0.0058 | 0.0054 | 0.0244 | 0.1486 | 0.1117 | 0.0829 |
| threonine | 0.8065 | 1.3198 | 1.4129 | 0.9008 | 1.0203 | 0.0731 | 0.0274 | 0.0342 | 0.0855 | 0.3148 | 0.0198 |
| aspartate | 0.7672 | 1.4093 | 1.5862 | 1.0045 | 0.9141 | 0.0355 | 0.0214 | 0.0282 | 0.0808 | 0.2643 | 0.3453 |
| asparagine | 0.6697 | 1.8954 | 2.1672 | 0.9084 | 0.9843 | 0.0076 | 0.0045 | 0.0207 | 0.0301 | 0.1137 | 0.0707 |
| beta-alanine | 1.1648 | 0.5442 | 0.6868 | 0.9173 | 1.452 | 0.013 | 0.0215 | 0.1395 | 0.0705 | 0.2034 | 0.2395 |
| Alanine | 0.7036 | 1.2541 | 1.2959 | 0.8075 | 1.0521 | 0.0016 | 0.0003 | 0.0166 | 0.22 | 0.2069 | 0.0317 |
| N-acetylalanine | 0.7541 | 0.9636 | 1.0992 | 0.8201 | 1.4087 | 0.2764 | 0.1682 | 0.4775 | 0.0391 | 0.82 | 0.0561 |
| glutamate | 0.7 | 1.0877 | 1.3387 | 0.7627 | 1.0169 | 0.0295 | 0.0267 | 0.152 | 0.2446 | 0.6849 | 0.0633 |
| gamma-aminobutyrate (GABA) | 0.9162 | 1.5757 | 2.3001 | 0.1343 | 0.1888 | 0.2833 | 0.013 | 0.0088 | 0.0271 | 0.0034 | 0.0628 |
| trans-urocanate | 0.7139 | 1.1611 | 1.1709 | 1.0096 | 0.8661 | 0.0073 | 0.0324 | 0.3286 | 0.0441 | 0.1358 | 0.309 |
| histamine | 0.1302 | 1.8813 | 1.8631 | 0.1332 | 0.1152 | 0.0215 | 0.0203 | 0.0173 | 0.024 | 0.921 | 0.4226 |
| imidazole propionate | 1.4837 | 1.1319 | 1.6681 | 0.8095 | 0.7836 | 0.34 | 0.9224 | 0.0483 | 0.104 | 0.075 | 0.062 |
| Lysine | 0.4414 | 1.5209 | 1.9933 | 0.6511 | 1.0092 | 0.0179 | 0.0002 | 0.0687 | 0.0518 | 0.0133 | 0.0044 |
| phenyllactate (PLA) | 1.3051 | 1.3164 | 2.626 | 0.8446 | 0.8676 | 0.9562 | 0.0621 | 0.3343 | 0.015 | 0.2344 | 0.2561 |
| phenylalanine | 0.6568 | 1.3946 | 1.5368 | 0.8743 | 1.0051 | 0.0179 | 0.004 | 0.0279 | 0.0816 | 0.1281 | 0.0349 |
| tyrosine | 0.627 | 1.3626 | 1.4481 | 0.8157 | 0.9221 | 0.0121 | 0.0027 | 0.0277 | 0.0941 | 0.2152 | 0.0287 |

| | Table 2b. Amino Acids and their Metabolites | | | | | | | | | | |
|---|---|---|---|---|---|---|---|---|---|---|---|
| | **Mean Values** | | | | | **Statistical P Values** | | | | | |
| **Biochemical Name** | **NON-Dan BASELINE** | **DANDRUFF BASELINE ZPT** | **DANDRUFF BASELINE Sel** | **WEEK 3 TRT ZPT** | **WEEK 3 TRT Sel** | **Dan Baseline ZPT vs Non Dan Baseline** | **Dan Baseline Sel vs Non Dan Baseline** | **Dan ZPT W3 vs Dan ZPT Baseline** | **Dan Sel W3 vs Dan Sel Baseline** | **Dan ZPT W3 vs Non Dan Baseline** | **Dan Sel W3 vs Non Dan Baseline** |
| 3-(4-hydroxyphenyl)lactate | 0.9412 | 1.0864 | 1.797 | 0.7551 | 0.7878 | 0.5295 | 0.049 | 0.0866 | 0.007 | 0.3337 | 0.4689 |
| tryptophan | 0.6623 | 1.3686 | 1.5271 | 0.846 | 0.9959 | 0.023 | 0.0063 | 0.0424 | 0.1528 | 0.1369 | 0.0193 |
| isoleucine | 0.6947 | 1.2904 | 1.3776 | 0.881 | 1.037 | 0.0504 | 0.0128 | 0.0359 | 0.2572 | 0.1602 | 0.0147 |
| Leucine | 0.6647 | 1.5007 | 1.624 | 0.8952 | 1.0377 | 0.0248 | 0.0005 | 0.0442 | 0.0695 | 0.022 | 0.0108 |
| Valine | 0.7966 | 1.2531 | 1.3776 | 0.9159 | 1.047 | 0.0508 | 0.0187 | 0.0765 | 0.2336 | 0.3468 | 0.0439 |
| cysteine | 0.6907 | 0.9516 | 1.0766 | 0.6907 | 0.9718 | 0.0261 | 0.1414 | 0.0261 | 0.749 | | 0.0171 |
| methionine sulfoxide | 0.9967 | 2.0122 | 1.9455 | 0.8522 | 0.8924 | 0.2847 | 0.0427 | 0.033 | 0.0416 | 0.5306 | 0.4235 |
| methionine | 0.7726 | 1.2609 | 1.4097 | 0.8953 | 1.0791 | 0.0722 | 0.0072 | 0.084 | 0.2313 | 0.256 | 0.0923 |
| N-acetylmethionine | 0.4387 | 1.7158 | 1.93 | 0.6395 | 0.5476 | 0.0147 | 0.0059 | 0.0021 | 0.0332 | 0.1103 | 0.2651 |
| dimethylarginine (SDMA + ADMA) | 0.9514 | 1.6824 | 1.6544 | 1.0243 | 0.8338 | 0.206 | 0.1848 | 0.1502 | 0.0603 | 0.6771 | 0.9739 |
| arginine | 0.8517 | 1.2968 | 1.356 | 0.7566 | 1.1244 | 0.0984 | 0.0485 | 0.0771 | 0.3125 | 0.5235 | 0.1471 |
| ornithine | 0.6531 | 0.9307 | 1.0937 | 1.6336 | 0.9562 | 0.0704 | 0.0545 | 0.0425 | 0.6237 | 0.0038 | 0.0569 |
| urea | 0.7055 | 0.6403 | 0.9078 | 2.6469 | 2.5539 | 0.6004 | 0.2738 | 0.1554 | 0.0632 | 0.1256 | 0.0055 |
| proline | 0.7636 | 1.3843 | 1.5126 | 0.9018 | 0.907 | 0.0415 | 0.0029 | 0.007 | 0.0315 | 0.3301 | 0.1239 |
| citrulline | 0.9275 | 1.3028 | 1.3029 | 0.9112 | 0.8749 | 0.1407 | 0.1687 | 0.0227 | 0.0531 | 0.8342 | 0.5529 |
| N-acetylornithine | 1.3412 | 0.8012 | 1.2805 | 0.964 | 0.7412 | 0.0424 | 0.741 | 0.4786 | 0.0624 | 0.1579 | 0.0549 |
| putrescine | 0.7016 | 2.179 | 3.8931 | 0.2637 | 0.4951 | 0.2133 | 0.038 | 0.0902 | 0.0087 | 0.2064 | 0.5501 |

| **Table 3a. Dipeptide Metabolites/ Biomarkers** | **Mean Values** | | | | | **Statistical P Values** | | | | | |
|---|---|---|---|---|---|---|---|---|---|---|---|
| | **NON-Dan BASELINE** | **DANDRUFF BASELINE ZPT** | **DANDRUFF BASELINE Sel** | **WEEK 3 TRT ZPT** | **WEEK 3 TRT Sel** | | | | | | |
| **Biochemical Name** | | | | | | **Dan Baseline ZPT vs Non Dan Baseline** | **Dan Baseline Sel vs Non Dan Baseline** | **Dan ZPT W3 vs Dan ZPT Baseline** | **Dan Sel W3 vs Dan Sel Baseline** | **Dan ZPT W3 vs Non Dan Baseline** | **Dan Sel W3 vs Non Dan Baseline** |
| glycylglycine | 0.7249 | 1.2799 | 1.3745 | 0.8857 | 1.0077 | 0.0482 | 0.0117 | 0.0806 | 0.2075 | 0.1978 | 0.1088 |
| glycylproline | 0.7622 | 1.2366 | 1.3936 | 0.7738 | 1.0808 | 0.0239 | 0.0348 | 0.0429 | 0.1276 | 0.9713 | 0.0242 |
| glycylisoleucine | 0.6565 | 1.0264 | 1.1623 | 1.0235 | 1.0373 | 0.0738 | 0.0226 | 0.9444 | 0.6414 | 0.0299 | 0.0298 |
| glycylleucine | 0.6045 | 1.0796 | 1.2576 | 0.9455 | 1.1588 | 0.0278 | 0.0198 | 0.3838 | 0.6424 | 0.0129 | 0.0085 |
| glycyltyrosine | 0.6439 | 0.9877 | 1.0941 | 0.8302 | 0.9502 | 0.155 | 0.0645 | 0.3585 | 0.2875 | 0.238 | 0.1135 |
| alanylhistidine | 0.7563 | 1.0494 | 1.2166 | 0.9266 | 1.0491 | 0.2886 | 0.0847 | 0.622 | 0.3493 | 0.3718 | 0.0503 |
| alanylphenylalanine | 0.5555 | 0.9178 | 1.4924 | 0.7004 | 1.1886 | 0.0917 | 0.0415 | 0.0509 | 0.5226 | 0.2764 | 0.0025 |
| alanyltyrosine | 0.6934 | 0.9572 | 1.1387 | 0.7214 | 1.179 | 0.1334 | 0.0503 | 0.222 | 0.9373 | 0.4226 | 0.0857 |
| arginylproline | 0.768 | 1.2404 | 1.6401 | 0.674 | 1.0015 | 0.1512 | 0.0223 | 0.1584 | 0.0311 | 0.5937 | 0.3352 |
| pyroglutamylvaline | 0.804 | 1.1774 | 1.2627 | 0.9039 | 0.9128 | 0.0347 | 0.0388 | 0.1558 | 0.1 | 0.4948 | 0.2969 |
| valylserine | 0.7273 | 1.3502 | 1.6873 | 0.8809 | 1.0209 | 0.0053 | 0.0067 | 0.1251 | 0.0568 | 0.3436 | 0.0345 |
| valylvaline | 0.8097 | 1.0987 | 1.6838 | 0.8111 | 0.802 | 0.2718 | 0.069 | 0.371 | 0.0858 | 0.9756 | 0.8781 |
| isoleucylglycine | 0.7582 | 1.1653 | 1.3376 | 0.9951 | 1.0283 | 0.0756 | 0.0347 | 0.3352 | 0.3278 | 0.2176 | 0.0908 |
| isoleucylserine | 0.7566 | 1.4226 | 1.4737 | 0.9003 | 1.1194 | 0.0721 | 0.0473 | 0.2745 | 0.4028 | 0.3294 | 0.0491 |
| leucylglutamate | 0.9421 | 0.7754 | 1.1844 | 0.874 | 0.9581 | 0.4915 | 0.6143 | 0.778 | 0.0361 | 0.7499 | 0.9563 |
| leucylglycine | 0.6008 | 1.2196 | 1.229 | 0.9929 | 1.0003 | 0.0487 | 0.033 | 0.5224 | 0.3485 | 0.0117 | 0.0226 |
| leucylserine | 0.5864 | 1.1371 | 1.2377 | 0.914 | 1.21 | 0.0625 | 0.1501 | 0.491 | 0.9588 | 0.0345 | 0.0488 |
| leucylvaline | 1.1083 | 0.6821 | 0.6891 | 1.1401 | 1.4937 | 0.0164 | 0.0196 | 0.0278 | 0.0719 | 0.8814 | 0.1268 |
| threonylleucine | 0.9091 | 1.0188 | 1.3876 | 0.7915 | 1.0105 | 0.6114 | 0.0447 | 0.4565 | 0.1222 | 0.4964 | 0.8045 |
| valylisoleucine | 0.8958 | 1.1007 | 1.303 | 0.9598 | 0.9589 | 0.3177 | 0.0983 | 0.0317 | 0.2208 | 0.692 | 0.3726 |
| serylisoleucine* | 0.9746 | 1.0649 | 1.1156 | 0.9496 | 1.1161 | 0.3969 | 0.3092 | 0.0623 | 0.9836 | 0.8071 | 0.2442 |
| valylglycine | 0.8876 | 1.0829 | 1.2064 | 0.9728 | 0.9123 | 0.3686 | 0.2296 | 0.5055 | 0.0748 | 0.5787 | 0.8041 |
| valylhistidine | 0.6803 | 1.1645 | 1.417 | 0.8434 | 0.9362 | 0.077 | 0.0582 | 0.3256 | 0.2325 | 0.2102 | 0.0487 |

**Table 3b Dipeptide Metabolites/Biomarkers**

| **Biochemical Name** | **Mean Values** | | | | | **Statistical P Values** | | | | | |
|---|---|---|---|---|---|---|---|---|---|---|---|
| | **NON-Dan BASELINE** | **DANDRUFF BASELINE ZPT** | **DANDRUFF BASELINE Sel** | **WEEK 3 TRT ZPT** | **WEEK 3 TRT Sel** | **Dan Baseline ZPT vs Non Dan Baseline** | **Dan Baseline Sel vs Non Dan Baseline** | **Dan ZPT W3 vs Dan ZPT Baseline** | **Dan Sel W3 vs Dan Sel Baseline** | **Dan ZPT W3 vs Non Dan Baseline** | **Dan Sel W3 vs Non Dan Baseline** |
| gamma-glutamylvaline | 0.82 | 1.2546 | 1.4437 | 1.0807 | 1.0515 | 0.0471 | 0.0081 | 0.446 | 0.1716 | 0.2345 | 0.2147 |
| gamma-glutamylleucine | 0.605 | 1.2635 | 1.5188 | 0.9572 | 0.8623 | 0.0093 | 0.0051 | 0.1751 | 0.0224 | 0.0447 | 0.0966 |
| gamma-glutamylisoleucine* | 0.6477 | 1.189 | 1.4317 | 1.0646 | 1.0435 | 0.0102 | 0.0017 | 0.4595 | 0.0378 | 0.0397 | 0.0099 |
| gamma-glutamylglutamate | 0.861 | 0.9495 | 1.011 | 1.7984 | 0.9586 | 0.5203 | 0.5657 | 0.0407 | 0.7417 | 0.0276 | 0.72 |
| gamma-glutamylphenylalanine | 0.7105 | 1.034 | 1.2919 | 0.9609 | 0.9352 | 0.1899 | 0.0881 | 0.6429 | 0.0097 | 0.261 | 0.2851 |
| gamma-glutamyltyrosine | 0.6735 | 1.1789 | 1.4397 | 1.0565 | 0.9048 | 0.0149 | 0.0066 | 0.5249 | 0.0517 | 0.0778 | 0.0838 |
| gamma-glutamylthreonine* | 0.8194 | 1.0463 | 1.2284 | 0.9414 | 0.716 | 0.1001 | 0.0282 | 0.4856 | 0.0283 | 0.6186 | 0.4486 |

**Table 4. Nucleic acid metabolites/Biomarkers**

| | **Mean Values** | | | | | **Statistical P Values** | | | | | |
|---|---|---|---|---|---|---|---|---|---|---|---|
| | **NON-Dan BASELINE** | **DANDRUFF BASELINE ZPT** | **DANDRUFF BASELINE Sel** | **WEEK 3 TRT ZPT** | **WEEK 3 TRT Sel** | **Dan Baseline ZPT vs Non Dan Baseline** | **Dan Baseline Sel vs Non Dan Baseline** | **Dan ZPT W3 vs Dan ZPT Baseline** | **Dan Sel W3 vs Dan Sel Baseline** | **Dan ZPT W3 vs Non Dan Baseline** | **Dan Sel W3 vs Non Dan Baseline** |
| **Biochemical Name** | | | | | | | | | | | |
| xanthine | 0.2702 | 3.7077 | 4.313 | 0.5918 | 0.6403 | 0.0007 | 0.0007 | 0.0157 | 0.0234 | 0.2696 | 0.071 |
| hypoxanthine | 0.4898 | 3.8435 | 4.3286 | 0.8173 | 0.9561 | 0.0029 | 0.0094 | 0.0068 | 0.0294 | 0.0018 | 0.0052 |
| inosine | 0.4048 | 1.1474 | 1.2437 | 0.4048 | 0.4208 | 0.037 | 0.0328 | 0.037 | 0.0459 | | 0.4226 |
| adenosine | 0.7407 | 1.2194 | 1.4535 | 0.5221 | 0.6054 | 0.1426 | 0.0655 | 0.0117 | 0.0099 | 0.2993 | 0.5538 |
| N1-methyladenosine | 0.5229 | 1.0577 | 1.2773 | 0.4038 | 0.4038 | 0.0632 | 0.0406 | 0.0032 | 0.0352 | 0.4226 | 0.4226 |
| guanine | 0.7073 | 2.4778 | 2.8086 | 0.4662 | 0.7571 | 0.0339 | 0.0918 | 0.0381 | 0.042 | 0.3354 | 0.8788 |
| guanosine | 0.6364 | 1.6641 | 1.8884 | 0.6943 | 0.7695 | 0.0031 | 0.0153 | 0.0307 | 0.086 | 0.6714 | 0.505 |
| allantoin | 0.6842 | 0.9112 | 2.4147 | 0.6255 | 1.101 | 0.5146 | 0.0696 | 0.0614 | 0.0108 | 0.9125 | 0.3114 |
| cytidine | 0.311 | 2.0225 | 2.2373 | 0.4321 | 0.503 | 0.0159 | 0.0001 | 0.0038 | 0.0237 | 0.5041 | 0.1242 |
| uridine | 0.964 | 2.0595 | 2.1484 | 0.8522 | 0.9172 | 0.0004 | 0.0096 | 0.0068 | 0.028 | 0.0556 | 0.4875 |
| N1-Methyl-2-pyridone-5-carboxamide | 1.5393 | 0.7485 | 0.9253 | 1.1768 | 0.8271 | 0.0128 | 0.1055 | 0.3365 | 0.7993 | 0.3763 | 0.0408 |

| **Table 5a. Lipid Biomarkers** | **Mean Values** | | | | | **Statistical P Values** | | | | | |
|---|---|---|---|---|---|---|---|---|---|---|---|
| **Biochemical Name** | **NON-Dan BASEL INE** | **DANDRUFF BASELINE ZPT** | **DANDRUFF BASELINE Sel** | **WEEK 3 TRT ZPT** | **WEEK 3 TRT Sel** | **Dan Baseline ZPT vs Non Dan Baseline** | **Dan Baseline Sel vs Non Dan Baseline** | **Dan ZPT W3 vs Dan ZPT Baseline** | **Dan Sel W3 vs Dan Sel Baseline** | **Dan ZPT W3 vs Non Dan Baseline** | **Dan Sel W3 vs Non Dan Baseline** |
| **linoleate (18:2n6)** | 0.9822 | 2.4078 | 1.5742 | 0.4272 | 0.3408 | 0.2769 | 0.0362 | 0.135 | 0.0054 | 0.0162 | 0.004 |
| **linolenate [alpha or gamma; (18:3n3 or 6)]** | 1.2755 | 1.0545 | 1.0125 | 0.8941 | 0.6753 | 0.3128 | 0.2565 | 0.5529 | 0.0645 | 0.0871 | 0.012 |
| **myristate (14:0)** | 1.5169 | 1.0431 | 1.1241 | 0.9113 | 0.6774 | 0.0481 | 0.1235 | 0.2309 | 0.0216 | 0.0251 | 0.0041 |
| **myristoleate (14:1 n5)** | 1.3848 | 1.0366 | 1.1899 | 0.8341 | 0.6261 | 0.14 | 0.4985 | 0.0617 | 0.0643 | 0.0342 | 0.0148 |
| **pentadecanoate (15:0)** | 1.5878 | 1.4592 | 1.6394 | 0.5867 | 0.3057 | 0.8255 | 0.8547 | 0.0249 | 0.0077 | 0.0431 | 0.0111 |
| **palmitate (16:0)** | 1.6437 | 0.9963 | 1.14 | 0.9476 | 0.6888 | 0.0843 | 0.0904 | 0.6866 | 0.0043 | 0.033 | 0.0055 |
| **palmitoleate (16:1 n7)** | 1.4662 | 1.0235 | 1.1443 | 0.8507 | 0.6347 | 0.062 | 0.1946 | 0.0016 | 0.0021 | 0.0198 | 0.0124 |
| **margarate (17:0)** | 1.5336 | 1.1631 | 1.2433 | 0.6953 | 0.3903 | 0.3683 | 0.4726 | 0.0905 | 0.0069 | 0.0437 | 0.0076 |
| **10-heptadecenoate (17:1 n7)** | 1.4182 | 1.1125 | 1.2435 | 0.7411 | 0.4139 | 0.3351 | 0.5997 | 0.0987 | 0.0103 | 0.0567 | 0.0228 |
| **stearate (18:0)** | 1.5209 | 0.9978 | 1.0168 | 1.0238 | 0.7856 | 0.0638 | 0.0522 | 0.9096 | 0.1449 | 0.1046 | 0.0157 |
| **oleate (18:1 n9)** | 1.4085 | 1.3481 | 1.2722 | 0.6901 | 0.4172 | 0.884 | 0.6741 | 0.0892 | 0.0109 | 0.0282 | 0.0202 |
| **nonadecanoate (19:0)** | 1.3859 | 1.1598 | 1.2544 | 0.5421 | 0.3608 | 0.5266 | 0.7546 | 0.0482 | 0.002 | 0.0235 | 0.0084 |
| **10-nonadecenoate (19:1n9)** | 1.0868 | 1.332 | 1.3744 | 0.4088 | 0.1901 | 0.4046 | 0.4111 | 0.0226 | 0.0069 | 0.038 | 0.0129 |
| **arachidate (20:0)** | 1.3768 | 1.4058 | 1.4706 | 0.7662 | 0.6154 | 0.9306 | 0.7107 | 0.0147 | 0.0127 | 0.0956 | 0.0534 |
| **eicosenoate (20:1 n9 or 11)** | 1.034 | 1.3159 | 1.2895 | 0.3561 | 0.202 | 0.394 | 0.4233 | 0.0188 | 0.0079 | 0.0551 | 0.0138 |
| **dihomo-linoleate (20:2n6)** | 1.2464 | 1.3338 | 1.442 | 0.3984 | 0.214 | 0.6804 | 0.5998 | 0.0187 | 0.0073 | 0.0461 | 0.0161 |
| **arachidonate (20:4n6)** | 1.1934 | 1.0367 | 1.2364 | 0.5815 | 0.3665 | 0.7521 | 0.8581 | 0.0067 | 0.0243 | 0.1046 | 0.0415 |
| **docosadienoate (22:2n6)** | 1.0854 | 1.2361 | 1.2483 | 0.3022 | 0.1554 | 0.5987 | 0.5631 | 0.0331 | 0.0061 | 0.0288 | 0.0076 |
| **docosatrienoate (22:3n3)** | 1.7654 | 2.1934 | 2.0368 | 0.5705 | 0.2735 | 0.4795 | 0.7183 | 0.0078 | 0.0013 | 0.1125 | 0.0233 |
| **2-hydroxypalmitate** | 1.3789 | 0.7926 | 1.1798 | 0.8231 | 0.6696 | 0.2217 | 0.26 | 0.5973 | 0.0062 | 0.0367 | 0.0044 |
| **13-HODE + 9-HODE** | 0.8777 | 1.6932 | 1.2389 | 0.4413 | 0.4182 | 0.2282 | 0.1635 | 0.0824 | 0.0067 | 0.054 | 0.0437 |
| ***oleamide*** | 5.0637 | 0.3294 | 0.7194 | 1.3968 | 1.1816 | 0.085 | 0.1668 | 0.0017 | 0.1677 | 0.361 | 0.2796 |
| **11-methyllauric acid** | 1.0735 | 1.7493 | 1.8851 | 0.52 | 0.4847 | 0.2238 | 0.1777 | 0.0098 | 0.0717 | 0.0779 | 0.0497 |
| **12-methyltridecanoic acid** | 1.1243 | 1.3351 | 1.4782 | 0.3858 | 0.2472 | 0.3342 | 0.1974 | 0.0046 | 0.0136 | 0.0122 | 0.0059 |
| **13-methylmyristic acid** | 1.2369 | 1.0466 | 1.4121 | 0.3878 | 0.2946 | 0.3803 | 0.451 | 0.0135 | 0.0149 | 0.0033 | 0.0125 |
| **15-methylpalmitate** | 1.3879 | 1.1998 | 1.2305 | 0.485 | 0.3813 | 0.6301 | 0.7187 | 0.0319 | 0.0043 | 0.0354 | 0.0152 |
| **isopalmitic acid** | 1.2561 | 1.2542 | 1.3342 | 0.5169 | 0.3034 | 0.9817 | 0.6521 | 0.0009 | 0.0116 | 0.0206 | 0.0046 |
| **17-methylstearate** | 1.2687 | 1.224 | 1.2798 | 0.4446 | 0.273 | 0.9792 | 0.8512 | 0.0243 | 0.0073 | 0.0234 | 0.0057 |

| Table 5b. Lipid Biomarkers | **Mean Values** | | | | | **Statistical P Values** | | | | | |
|---|---|---|---|---|---|---|---|---|---|---|---|
| **Biochemical Name** | **NON-Dan BASEL INE** | **DANDRUF F BASELINE ZPT** | **DANDRUFF BASELINE Sel** | **WEEK 3 TRT ZPT** | **WEEK 3 TRT Sel** | **Dan Baseline ZPT vs Non Dan Baseline** | **Dan Baseline Sel vs Non Dan Baseline** | **Dan ZPT W3 vs Dan ZPT Baseline** | **Dan Sel W3 vs Dan Sel Baseline** | **Dan ZPT W3 vs Non Dan Baseline** | **Dan Sel W3 vs Non Dan Baseline** |
| **19-methylarachidic acid** | 1.4755 | 0.8952 | 1.5477 | 0.8865 | 0.7113 | 0.0499 | 0.9171 | 0.6743 | 0.0521 | 0.2033 | 0.0299 |
| **2-methylpalmitate** | 1.0341 | 1.1748 | 1.4369 | 0.2049 | 0.1469 | 0.5191 | 0.0318 | 0.0038 | 0.0007 | 0.0145 | 1.05E-05 |
| **oleic ethanolamide** | 0.9814 | 0.9804 | 0.665 | 1.5447 | 1.1213 | 0.785 | 0.0662 | 0.3672 | 0.1121 | 0.1019 | 0.6213 |
| **palmitoyl ethanolamide** | 0.7992 | 1.1504 | 0.6282 | 3.5409 | 4.2036 | 0.5716 | 0.1894 | 0.1073 | 0.0064 | 0.0008 | 0.0006 |
| **stearoyl ethanolamide** | 0.6043 | 0.7969 | 0.5296 | 2.3466 | 2.6645 | 0.461 | 0.6221 | 0.1642 | 0.0009 | 0.0192 | 0.004 |
| **1-octadecanol** | 3.5297 | 0.7195 | 0.8975 | 5.2198 | 1.873 | 0.0572 | 0.104 | 0.1762 | 0.5585 | 0.9295 | 0.3329 |
| **1-hexadecanol** | 1.4398 | 1.1639 | 1.2711 | 0.9185 | 0.3847 | 0.6395 | 0.828 | 0.1257 | 0.0158 | 0.2814 | 0.0418 |
| **Ethanolamine** | 0.3853 | 0.9076 | 1.0604 | 0.976 | 1.7285 | 0.0146 | 0.0113 | 0.8888 | 0.0518 | 0.0625 | 0.0123 |
| **diethanolamine** | 1.7641 | 1.1706 | 0.7364 | 1.0654 | 1.3489 | 0.4694 | 0.1794 | 0.7726 | 0.0171 | 0.4142 | 0.7145 |
| **Glycerol** | 0.9243 | 2.1907 | 3.0179 | 0.8763 | 0.5157 | 0.0016 | 0.0229 | 0.0357 | 0.0091 | 0.711 | 0.0549 |
| **glycerol 3-phosphate (G3P)** | 0.5474 | 1.064 | 1.4683 | 0.5943 | 0.8007 | 0.1432 | 0.0698 | 0.1908 | 0.3264 | 0.9528 | 0.6975 |
| **myo-inositol** | 0.9946 | 1.0907 | 1.2808 | 0.8027 | 0.844 | 0.6432 | 0.3678 | 0.3439 | 0.0874 | 0.6716 | 0.7679 |
| **1-myristoylglycerol (1-monomyristin)** | 1.02 | 1.022 | 1.4218 | 0.9563 | 0.6055 | 0.7933 | 0.4877 | 0.6386 | 0.0245 | 0.9691 | 0.3012 |
| **1-pentadecanoylglycerol (1-monopentadecanoin)** | 1.0693 | 1.1071 | 1.3066 | 1.0552 | 0.5786 | 0.7381 | 0.6835 | 0.6696 | 0.0216 | 0.9281 | 0.1718 |
| **1-heptadecanoylglycerol (1-monoheptadecanoin)** | 0.9261 | 1.015 | 1.0472 | 1.1274 | 0.638 | 0.6342 | 0.6608 | 0.4169 | 0.0859 | 0.4913 | 0.5644 |
| **2-palmitoylglycerol (2-monopalmitin)** | 1.0303 | 0.6772 | 0.9083 | 1.0617 | 1.2351 | 0.3007 | 0.7521 | 0.0415 | 0.0624 | 0.7684 | 0.5595 |
| **1-stearoylglycerol (1-monostearin)** | 1.0932 | 0.4353 | 0.5667 | 1.1254 | 1.2751 | 0.0097 | 0.0317 | 0.0344 | 0.0099 | 0.8715 | 0.138 |
| **2-stearoylglycerol (2-monostearin)** | 1.077 | 0.6802 | 0.7315 | 1.1493 | 1.4177 | 0.1038 | 0.0288 | 0.0429 | 0.0305 | 0.5624 | 0.1077 |
| **1-oleoylglycerol (1-monoolein)** | 1.0685 | 1.1838 | 1.1727 | 0.7432 | 0.5487 | 0.6011 | 0.8415 | 0.0901 | 0.0073 | 0.3882 | 0.1728 |
| **Sphingosine** | 0.8863 | 0.8766 | 0.8238 | 1.5997 | 2.017 | 0.985 | 0.7518 | 0.1325 | 0.0504 | 0.1109 | 0.0267 |
| **Lathosterol** | 0.7065 | 1.1156 | 1.047 | 0.7479 | 0.9469 | 0.0824 | 0.1193 | 0.0716 | 0.6959 | 0.8922 | 0.2332 |
| **7-al pha-hydroxycholesterol** | 1.4839 | 0.7595 | 1.0855 | 0.959 | 0.9662 | 0.0837 | 0.445 | 0.2919 | 0.9267 | 0.2203 | 0.3382 |

**Table 6. Miscellaneous including carbohydrates and their metabolites, co-factors and others**

| | **Mean Value** | | | | | **Statistical P Value** | | | | | |
|---|---|---|---|---|---|---|---|---|---|---|---|
| **Biochemical Name** | **NON-Dan BASELINE** | **DANDRUFF BASELINE ZPT** | **DANDRUFF BASELINE Sel** | **WEEK 3 TRT ZPT** | **WEEK 3 TRT Sel** | | | | | | |
| | | | | | | **Dan Baseline ZPT vs Non Dan Baseline** | **Dan Baseline Sel vs Non Dan Baseline** | **Dan ZPT W3 vs Dan ZPT Baseline** | **Dan Sel W3 vs Dan Sel Baseline** | **Dan ZPT W3 vs Non Dan Baseline** | **Dan Sel W3 vs Non Dan Baseline** |
| **fructose** | 3.923 | 0.9113 | 1.1783 | 0.9183 | 0.7647 | 0.1807 | 0.2528 | 0.9928 | 0.0961 | 0.1796 | 0.1264 |
| **mannitol** | 1.4139 | 1.4653 | 2.4459 | 0.3709 | 0.1067 | 0.8797 | 0.1526 | 0.0713 | 0.001 | 0.1027 | 0.0012 |
| **trehalose** | 2.0073 | 0.8953 | 1.7628 | 1.0379 | 0.6348 | 0.1317 | 0.8258 | 0.839 | 0.0265 | 0.2037 | 0.0725 |
| **glucose** | 2.8799 | 0.9035 | 1.0642 | 0.9182 | 0.7621 | 0.0886 | 0.1319 | 0.9401 | 0.0322 | 0.0963 | 0.0746 |
| **pyruvate** | 0.928 | 0.5048 | 0.7472 | 2.5745 | 3.2739 | 0.2183 | 0.3238 | 0.0538 | 0.0005 | 0.1863 | 0.0021 |
| **lactate** | 0.8089 | 0.6938 | 0.7137 | 2.7727 | 3.8436 | 0.5162 | 0.4966 | 0.0452 | 0.0287 | 0.1027 | 0.0061 |
| **arabitol** | 1.0021 | 1.0611 | 1.4634 | 0.6515 | 1.0705 | 0.7411 | 0.3132 | 0.0295 | 0.4702 | 0.4092 | 0.7317 |
| **ribitol** | 0.6913 | 1.5209 | 2.9087 | 0.2014 | 0.2485 | 0.0953 | 0.0445 | 0.0155 | 0.0026 | 0.001 | 0.023 |
| **gluconate** | 2.9075 | 1.0593 | 1.4443 | 0.84 | 0.7319 | 0.511 | 0.7628 | 0.2328 | 0.0957 | 0.3792 | 0.3036 |
| **pantothenate** | 0.5884 | 1.1679 | 1.4267 | 0.3417 | 0.3417 | 0.0677 | 0.0009 | 0.0248 | 0.0025 | 0.0088 | 0.0088 |
| **fumarate** | 1.1599 | 1.4737 | 2.367 | 0.786 | 0.8853 | 0.5372 | 0.0218 | 0.2751 | 0.0599 | 0.1666 | 0.1585 |
| **malate** | 1.0384 | 2.3545 | 2.526 | 0.4613 | 0.4783 | 0.0419 | 0.0088 | 0.0012 | 0.0169 | 0.0213 | 0.0064 |
| **phosphate** | 0.8862 | 1.486 | 1.5802 | 0.9162 | 1.1108 | 0.0634 | 0.0069 | 0.1222 | 0.1444 | 0.8061 | 0.4488 |

**Table 7a and b. Structure unknown molecules/biomarkers**

| | | | | | | | | | | | |
|---|---|---|---|---|---|---|---|---|---|---|---|
| **Table 7a** | **Mean Values** | | | | | **Statistical P Values** | | | | | |
| | **NON-Dan BASELINE** | **DANDRUFF BASELINE ZPT** | **DANDRUFF BASELINE Sel** | **WEEK 3 TRT ZPT** | **WEEK 3 TRT Sel** | | | | | | |
| **Name** | | | | | | **Dan Baseline ZPT vs Non Dan Baseline** | **Dan Baseline Sel vs Non Dan Baseline** | **Dan ZPT W3 vs Dan Baseline** | **Dan Sel W3 vs Dan Sel Baseline** | **Dan ZPT W3 vs Non Dan Baseline** | **Dan Sel W3 vs Non Dan Baseline** |
| X - 11297 | 0.9666 | 0.5396 | 0.4803 | 1.3333 | 1.4949 | 0.0884 | 0.0005 | 0.1171 | 0.0336 | 0.161 | 0.1158 |
| X - 11509 | 1.4588 | 0.7392 | 0.7411 | 1.2137 | 1.1843 | 0.0496 | 0.0355 | 0.0077 | 0.2004 | 0.3056 | 0.1834 |
| X - 11533 | 0.9913 | 0.5204 | 0.5811 | 1.6566 | 1.5496 | 0.0367 | 0.01 | 0.0849 | 0.0012 | 0.3888 | 0.0107 |
| X - 11543 | 1.3947 | 0.7415 | 0.7987 | 1.2003 | 1.2273 | 0.0553 | 0.0667 | 0.0272 | 0.2203 | 0.2538 | 0.3537 |
| X - 12565 | 0.8925 | 1.1072 | 1.2566 | 0.8671 | 0.969 | 0.2029 | 0.1754 | 0.0038 | 0.0661 | 0.8295 | 0.6226 |
| X - 12776 | 1.2635 | 0.6168 | 0.8703 | 1.6819 | 2.866 | 0.1373 | 0.4167 | 0.077 | 0.0682 | 0.3404 | 0.108 |
| X - 13005 | 1.5466 | 0.5365 | 0.8165 | 1.574 | 0.9147 | 0.2231 | 0.4416 | 0.0115 | 0.3875 | 0.7128 | 0.5726 |
| X - 13081 | 1.0952 | 0.7407 | 0.4851 | 1.9015 | 2.3003 | 0.1961 | 0.0065 | 0.1183 | 0.002 | 0.2727 | 0.0208 |
| X - 13230 | 1.3128 | 0.4717 | 0.6354 | 1.1109 | 1.0725 | 0.033 | 0.0875 | 0.0621 | 0.1623 | 0.4225 | 0.3643 |
| X - 13372 | 1.4564 | 0.8324 | 0.8383 | 1.1143 | 1.1101 | 0.1033 | 0.056 | 0.1086 | 0.2632 | 0.1256 | 0.0202 |
| X - 13504 | 0.7943 | 1.1217 | 1.1245 | 0.9656 | 1.1986 | 0.009 | 0.1845 | 0.3377 | 0.4505 | 0.3896 | 0.0561 |
| X - 13529 | 0.7919 | 1.0476 | 1.2196 | 0.9748 | 1.0955 | 0.0512 | 0.0428 | 0.4211 | 0.1008 | 0.1887 | 0.0596 |
| X - 13582 | 1.0729 | 1.2129 | 1.1017 | 0.9817 | 0.7747 | 0.5132 | 0.86 | 0.3681 | 0.0009 | 0.6181 | 0.1202 |
| X - 13668 | 1.0788 | 1.5621 | 1.3078 | 0.6632 | 0.6146 | 0.2433 | 0.4757 | 0.1317 | 0.0494 | 0.0852 | 0.0044 |
| X - 13737 | 0.7407 | 1.3227 | 1.0491 | 0.4607 | 0.4713 | 0.1646 | 0.278 | 0.0402 | 0.0085 | 0.327 | 0.3492 |
| X - 13828 | 1.3513 | 0.6473 | 0.6187 | 1.195 | 1.2981 | 0.102 | 0.0324 | 0.0588 | 0.08 | 0.513 | 0.7674 |
| X - 14095 | 0.8486 | 1.2249 | 1.2882 | 0.937 | 0.9923 | 0.075 | 0.0892 | 0.0512 | 0.2623 | 0.4126 | 0.3841 |
| X - 14097 | 0.6749 | 1.3367 | 1.2752 | 0.9234 | 0.9185 | 0.0973 | 0.0149 | 0.0452 | 0.1132 | 0.3227 | 0.0356 |
| X - 14099 | 0.8806 | 1.1044 | 1.1911 | 0.9609 | 1.1999 | 0.095 | 0.4442 | 0.1376 | 0.8308 | 0.6828 | 0.1325 |
| X - 14196 | 0.8039 | 1.1794 | 1.239 | 0.9121 | 0.9871 | 0.0291 | 0.0833 | 0.031 | 0.3093 | 0.4225 | 0.1564 |
| X - 14198 | 0.7095 | 1.2557 | 1.4872 | 0.8929 | 1.1725 | 0.1368 | 0.0188 | 0.1462 | 0.0124 | 0.2843 | 0.0506 |
| X - 14302 | 0.7834 | 1.2364 | 1.3136 | 0.9264 | 0.948 | 0.0315 | 0.0269 | 0.0016 | 0.2996 | 0.3293 | 0.3013 |
| X - 14314 | 0.7265 | 1.3329 | 1.4244 | 0.9553 | 0.9849 | 0.028 | 0.0052 | 0.0349 | 0.1872 | 0.0767 | 0.0987 |
| X - 14427 | 0.9026 | 1.1968 | 1.424 | 1.017 | 1.0002 | 0.2083 | 0.0415 | 0.121 | 0.1898 | 0.6606 | 0.4297 |

| **Table 7b** | **NON-Dan BASELINE** | **DANDRUFF BASELINE ZPT** | **DANDRUFF BASELINE Sel** | **WEEK 3 TRT ZPT** | **WEEK 3 TRT Sel** | **Dan Baseline ZPT vs Non Dan Baseline** | **Dan Baseline Sel vs Non Dan Baseline** | **Dan ZPT W3 vs Dan ZPT Baseline** | **Dan Sel W3 vs Dan Sel Baseline** | **Dan ZPT W3 vs Non Dan Baseline** | **Dan Sel W3 vs Non Dan Baseline** |
|---|---|---|---|---|---|---|---|---|---|---|---|
| X - 14445 | 0.7404 | 1.2369 | 1.297 | 0.9104 | 0.9816 | 0.0606 | 0.0404 | 0.1944 | 0.2878 | 0.2926 | 0.1501 |
| X - 14904 | 0.7252 | 9.5657 | 2.0433 | 0.5071 | 0.6083 | 0.1671 | 0.049 | 0.1524 | 0.0092 | 0.4326 | 0.721 |
| X - 15559 | 0.9132 | 1.1629 | 0.5077 | 0.4525 | 0.3231 | 0.4493 | 0.4981 | 0.0828 | 0.4226 | 0.4349 | 0.2805 |
| X - 15562 | 0.8854 | 1.1182 | 0.4952 | 0.4293 | 0.309 | 0.4561 | 0.5115 | 0.0835 | 0.4226 | 0.4371 | 0.2903 |
| X - 15657 | 1.511 | 0.8866 | 0.7953 | 1.0636 | 1.1495 | 0.0919 | 0.0467 | 0.2788 | 0.2672 | 0.0455 | 0.1267 |
| X - 15664 | 1.6839 | 0.7156 | 0.6934 | 0.9554 | 1.3105 | 0.0146 | 0.0217 | 0.5711 | 0.2137 | 0.1281 | 0.2853 |
| X - 15689 | 0.8267 | 1.1844 | 0.6011 | 1.4769 | 1.3805 | 0.9614 | 0.6184 | 0.193 | 0.057 | 0.8047 | 0.3047 |
| X - 15782 | 1.4017 | 0.7368 | 0.7914 | 1.1374 | 1.1833 | 0.0412 | 0.0496 | 0.0365 | 0.157 | 0.2211 | 0.1642 |
| X - 15808 | 1.594 | 0.6448 | 0.6745 | 0.8948 | 1.5456 | 0.0663 | 0.0168 | 0.3034 | 0.0503 | 0.0361 | 0.9419 |
| X - 15863 | 1.101 | 1.0544 | 1.1629 | 0.921 | 0.8749 | 0.6967 | 0.9208 | 0.082 | 0.2241 | 0.2622 | 0.1345 |
| X - 16212 | 1.5842 | 0.825 | 0.9179 | 1.0924 | 1.0211 | 0.1043 | 0.0478 | 0.1825 | 0.5761 | 0.0982 | 0.0097 |
| X - 16468 | 0.8714 | 1.0576 | 1.247 | 0.8277 | 0.9406 | 0.4332 | 0.189 | 0.0097 | 0.1768 | 0.8315 | 0.7322 |
| X - 16626 | 1.7909 | 1.2852 | 0.8957 | 1.5567 | 1.4828 | 0.3739 | 0.0341 | 0.1341 | 0.1616 | 0.5553 | 0.5232 |
| X - 17375 | 1.4814 | 0.7667 | 0.8238 | 1.1529 | 1.205 | 0.0551 | 0.0485 | 0.0416 | 0.2002 | 0.168 | 0.1403 |
| X - 17553 | 2.0375 | 0.6632 | 0.8659 | 1.064 | 0.5404 | 0.0363 | 0.0702 | 0.1369 | 0.0609 | 0.1541 | 0.0194 |
| X - 17758 | 0.9163 | 1.5552 | 1.7789 | 0.7191 | 0.8423 | 0.1366 | 0.0902 | 0.073 | 0.0678 | 0.545 | 0.9176 |
| X - 17971 | 0.4506 | 1.9348 | 1.2308 | 0.2443 | 0.2586 | 0.0305 | 0.0701 | 0.0179 | 0.0092 | 0.3093 | 0.3531 |
| X -18111 | 1.3762 | 1.2473 | 0.738 | 1.3352 | 1.0941 | 0.5466 | 0.0539 | 0.3473 | 0.0948 | 0.7811 | 0.4537 |
| X - 18113 | 1.4317 | 1.3773 | 0.8558 | 1.6496 | 1.0511 | 0.5757 | 0.1201 | 0.202 | 0.0315 | 0.978 | 0.2738 |
| X - 18309 | 1.5985 | 1.5185 | 0.7675 | 1 .4394 | 1.2795 | 0.5421 | 0.0365 | 0.505 | 0.2881 | 0.6333 | 0.5039 |
| X - 18341 | 0.6642 | 1.1792 | 1.2903 | 0.9307 | 0.9762 | 0.028 | 0.0676 | 0.0978 | 0.2124 | 0.2453 | 0.0168 |

**Table 8. Self Assessment and Expert Grading Results**

| Sign/Symptom | Baseline ± STDERR | Post Treatment ± STDERR | Change from baseline p-value |
|---|---|---|---|
| Itch | 1.941 ± 0.096 | 0.778 ± 0.078 | <0.05 |
| Irritation | 0.240 ± 0.060 | 0.082 ± 0.028 | <0.05 |
| Dryness | 1.190 ± 0.113 | 0.752 ± 0.084 | <0.05 |
| Flaking | 2.467 ± 0.083 | 1.391 ± 0.072 | <0.05 |
| Flaking (expert grader) | | | |
| Dandruff | 29.57 ± 0.63 | 9.7 ± 0.76 | <0.05 |
| Non-Dandruff | 3.138 ± 0.76 | | |

## Claims

1. A noninvasive method for diagnosing dandruff in a subject comprising:
detecting a level of one or more small molecule biomarkers in the epithelial cell sample/skin cell sample that has been collected from the subject;
diagnosing the subject as having a dandruff based on the level of a detected small molecule biomarker, wherein the detected small molecule biomarker is uridine.

2. A method for diagnosing dandruff in a subject according to claim 1 comprising:
providing an adhesive article that has been applied to an epithelium of a mammal; has been allowed to adhere epithelial cells; and has been removed from the epithelium of the mammal;
preparing the adhesive article using standard laboratory methods for extraction;
extracting a small molecule biomarker from the epithelial cells adhered to said adhesive article;
measuring the small molecule biomarker from the epithelial cells adhered to said adhesive article;
determining the amount of the small molecule biomarker in the epithelial cells as compared to a baseline sample.

3. A method for diagnosing dandruff in a subject according to any preceding claims comprising:
detecting a level of one or more small molecule biomarkers in the epithelial cell sample/skin cell sample that has been collected from the subject;
comparing the level of detected small molecule biomarker in the epithelial cell sample to a small molecule biomarker reference level to thereby generate a differential level.

4. A method according to any preceding claims wherein the level of the detected small molecule biomarker is standardized by dividing the small molecule biomarker by an amount of protein on the adhesive article.

5. A method according to any preceding claims wherein there is a change in level in small molecule biomarker level when compared to a baseline level of small molecule biomarker.

6. A method according to any preceding claims wherein there is at least a 5% change from baseline in standardized small molecule biomarker following application with an antifungal hair treatment, when compared to a baseline level of small molecule biomarker prior to the application.

7. A method according to any preceding claims wherein there is at least a 5% reduction in standardized small molecule biomarker following application with an anti-dandruff shampoo when compared to a baseline level of small molecule biomarker prior to the application.

8. A method according to any preceding claims wherein there is at least a 5% reduction in standardized small molecule biomarker following application with a zinc pyrithione shampoo when compared to a baseline level of small molecule biomarker prior to the application.

9. A method according to any preceding claims wherein there is at least a 5% reduction in standardized small molecule biomarker following application with a selenium sulfide shampoo when compared to a baseline level of small molecule biomarker prior to the application.

10. A method according to any preceding claims wherein there is at least a 5% increase in standardized small molecule biomarker following application with an anti-dandruff shampoo when compared to a baseline level of small molecule biomarker prior to the application.

11. A method according to any preceding claims wherein there is at least a 5% increase in standardized small molecule biomarker following application with a zinc pyrithione shampoo when compared to a baseline level of small molecule biomarker prior to the application.

12. A method according to any preceding claims wherein there is at least a 5% increase in standardized small molecule biomarker following application with a selenium sulfide shampoo when compared to a baseline level of small molecule biomarker prior to the application.

13. A method according to any preceding claims wherein there is at least a 5% improvement in dandruff compared to a normal population.

14. A method of objectively measuring the perception of a symptom of dandruff in mammals said method comprising the steps of:
providing an adhesive article that has been applied to an epithelium of a mammal; has been allowed to adhere epithelial cells; and has been removed from the epithelium of the mammal;
preparing the adhesive article using standard laboratory methods for extraction;
extracting small molecule biomarker from the epithelial cells adhered to said adhesive article;
measuring small molecule biomarker from the epithelial cells adhered to said adhesive article and;
determining the amount of small molecule biomarker in the epithelial cells as compared to a baseline sample; wherein the small molecule biomarker is uridine.

15. A method according to any preceding claims wherein there is a reduction in small molecule biomarker from a baseline level which is directly proportional to a reduction in a symptom perception of dandruff.

## Patentansprüche

1. Nicht-invasives Verfahren zum Diagnostizieren von Schuppen bei einem Probanden, umfassend:
Nachweisen eines Niveaus von einem oder mehreren niedermolekularen Biomarkern in der Epithelzellprobe/Hautzellenprobe, die von dem Probanden entnommen wurde;
Diagnostizieren, dass der Proband an Schuppen leidet, basierend auf dem Niveau eines nachgewiesenen niedermolekularen Biomarkers, wobei das nachgewiesene niedermolekulare Molekül Uridin ist.

2. Verfahren zum Diagnostizieren von Schuppen bei einem Probanden nach Anspruch 1, umfassend:
Bereitstellen eines Klebstoffartikels, der auf ein Epithel eines Säugetiers aufgebracht wurde; wurde erlaubt, auf Epithelzellen zu haften; und wurde von dem Epithel des Säugetiers entfernt;
Vorbereiten des Klebstoffartikels mittels Standard-Laborverfahren zur Extraktion;
Extrahieren eines niedermolekularen Biomarkers aus den Epithelzellen, die an dem Klebstoffartikel haften;
Messen des niedermolekularen Biomarkers aus den Epithelzellen, die an dem Klebstoffartikel haften;
Bestimmen der Menge des niedermolekularen Biomarkers in den Epithelzellen im Vergleich zu einer Ausgangsprobe.

3. Verfahren zum Diagnostizieren von Schuppen bei einem Probanden nach einem der vorstehenden Ansprüche, umfassend:
Nachweisen eines Niveaus von einem oder mehreren niedermolekularen Biomarkern in der Epithelzellprobe/Hautzellenprobe, die von dem Probanden entnommen wurde;
Vergleichen des Niveaus des nachgewiesenen niedermolekularen Biomarkers in der Epithelzellenprobe mit einem Referenzniveau des niedermolekularen Biomarkers, um dadurch ein Differenzniveau zu erzeugen.

4. Verfahren nach einem der vorstehenden Ansprüche, wobei das Niveau des nachgewiesenen niedermolekularen Biomarkers durch Dividieren des niedermolekularen Biomarkers durch eine Proteinmenge an dem Klebstoffartikel standardisiert wird.

5. Verfahren nach einem der vorstehenden Ansprüche, wobei eine Änderung des Niveaus des niedermolekularen Biomarkers im Vergleich zu einem Ausgangsniveau des niedermolekularen Biomarkers vorliegt.

6. Verfahren nach einem der vorstehenden Ansprüche, wobei eine Änderung von mindestens 5 % vom Ausgangswert des standardisierten niedermolekularen Biomarkers nach Anwendung einer pilzbefallverhütenden Haarbehandlung im Vergleich zu einem Ausgangsniveau des niedermolekularen Biomarkers vor der Anwendung vorliegt.

7. Verfahren nach einem der vorstehenden Ansprüche, wobei eine Verringerung von mindestens 5 % des standardisierten niedermolekularen Biomarkers nach Anwendung eines Antischuppen-Shampoos im Vergleich zu einem Ausgangsniveau des niedermolekularen Biomarkers vor der Anwendung vorliegt.

8. Verfahren nach einem der vorstehenden Ansprüche, wobei eine Verringerung von mindestens 5 % des standardisierten niedermolekularen Biomarkers nach Anwendung eines Zink-Pyrithion-Shampoos im Vergleich zu einem Ausgangsniveau des niedermolekularen Biomarkers vor der Anwendung vorliegt.

9. Verfahren nach einem der vorstehenden Ansprüche, wobei eine Verringerung von mindestens 5 % des standardisierten niedermolekularen Biomarkers nach Anwendung eines Selensulfid-Shampoos im Vergleich zu einem Ausgangsniveau des niedermolekularen Biomarkers vor der Anwendung vorliegt.

10. Verfahren nach einem der vorstehenden Ansprüche, wobei eine Erhöhung von mindestens 5 % des standardisierten niedermolekularen Biomarkers nach Anwendung eines Antischuppen-Shampoos im Vergleich zu einem Ausgangsniveau des niedermolekularen Biomarkers vor der Anwendung vorliegt.

11. Verfahren nach einem der vorstehenden Ansprüche, wobei eine Erhöhung von mindestens 5 % des standardisierten niedermolekularen Biomarkers nach Anwendung eines Zink-Pyrithion-Shampoos im Vergleich zu einem Ausgangsniveau des niedermolekularen Biomarkers vor der Anwendung vorliegt.

12. Verfahren nach einem der vorstehenden Ansprüche, wobei eine Erhöhung von mindestens 5 % des standardisierten niedermolekularen Biomarkers nach Anwendung eines Selensulfid-Shampoos im Vergleich zu einem Ausgangsniveau des niedermolekularen Biomarkers vor der Anwendung vorliegt.

13. Verfahren nach einem der vorstehenden Ansprüche, wobei eine Verbesserung von mindestens 5 % bei Schuppen im Vergleich zu einer normalen Population vorliegt.

14. Verfahren zum objektiven Messen der Wahrnehmung eines Symptoms von Schuppen bei Säugetieren, wobei das Verfahren die folgenden Schritte umfasst:
Bereitstellen eines Klebstoffartikels, der auf ein Epithel eines Säugetiers aufgebracht wurde; wurde erlaubt, auf Epithelzellen zu haften; und wurde von dem Epithel des Säugetiers entfernt;
Vorbereiten des Klebstoffartikels mittels Standard-Laborverfahren zur Extraktion;
Extrahieren des niedermolekularen Biomarkers aus den Epithelzellen, die an dem Klebstoffartikel haften;
Messen des niedermolekularen Biomarkers aus den Epithelzellen, die an dem Klebstoffartikel haften, und;
Bestimmen der Menge des niedermolekularen Biomarkers in den Epithelzellen im Vergleich zu einer Ausgangsprobe; wobei der niedermolekulare Biomarker Uridin ist.

15. Verfahren nach einem der vorstehenden Ansprüche, wobei eine Reduzierung des niedermolekularen Biomarkers gegenüber einem Ausgangsniveau vorliegt, das direkt proportional zu einer Verringerung der Symptomwahrnehmung von Schuppen ist.

## Revendications

1. Procédé non-invasif de diagnostic de pellicules chez un sujet comprenant :
la détection d'un niveau d'un ou plusieurs biomarqueurs de petites molécules dans l'échantillon de cellules épithéliales / l'échantillon de cellules de peau qui a été collecté chez le sujet ;
le diagnostic du sujet comme ayant des pellicules en fonction du niveau d'un biomarqueur de petite molécule détecté, dans lequel la petite molécule détectée est l'uridine.

2. Procédé de diagnostic de pellicules chez un sujet selon la revendication 1 comprenant :
fournir un article adhésif qui a été appliqué à un épithélium d'un mammifère ; a été autorisé à adhérer à des cellules épithéliales ; et a été éliminé de l'épithélium du mammifère ;
préparer l'article adhésif en utilisant des procédés standard de laboratoire pour l'extraction ;
l'extraction d'un biomarqueur de petite molécule des cellules épithéliales mises en adhésion sur ledit article adhésif ;
la mesure du biomarqueur de petite molécule provenant des cellules épithéliales mises en adhésion sur ledit article adhésif ;
la détermination de la quantité du biomarqueur de petite molécule dans les cellules épithéliales par comparaison avec un échantillon de base.

3. Procédé de diagnostic de pellicules chez un sujet selon l'une quelconque des revendications précédentes comprenant :
la détection d'un niveau d'un ou plusieurs biomarqueurs de petites molécules dans l'échantillon de cellules épithéliales / l'échantillon de cellules de peau qui a été collecté chez le sujet ;
la comparaison du niveau de biomarqueur de petite molécule détecté dans l'échantillon de cellules épithéliales à une référence de biomarqueur de petite molécule afin de générer de ce fait un niveau différentiel.

4. Procédé selon l'une quelconque des revendications précédentes dans lequel le niveau du biomarqueur de petite molécule détecté est normalisé en divisant le biomarqueur de petite molécule par une quantité de protéine sur l'article adhésif.

5. Procédé selon l'une quelconque des revendications précédentes dans lequel il existe un changement du niveau de biomarqueur de petite molécule par comparaison avec un niveau de base du biomarqueur de petite molécule.

6. Procédé selon l'une quelconque des revendications précédentes dans lequel il existe un changement d'au moins 5 % par rapport à la base dans le biomarqueur normalisé de petite molécule à la suite d'une application avec un traitement capillaire antifongique, par comparaison avec un niveau de base du biomarqueur de petite molécule avant l'application.

7. Procédé selon l'une quelconque des revendications précédentes dans lequel il existe une réduction d'au moins 5 % du biomarqueur normalisé de petite molécule à la suite d'une application d'un shampooing anti-pelliculaire par comparaison avec un niveau de base de biomarqueur de petite molécule avant l'application.

8. Procédé selon l'une quelconque des revendications précédentes dans lequel il existe une réduction d'au moins 5 % du biomarqueur normalisé de petite molécule à la suite d'une application avec un shampooing à la pyrithione de zinc par comparaison avec un niveau de base de biomarqueur de petite molécule avant l'application.

9. Procédé selon l'une quelconque des revendications précédentes dans lequel il existe une réduction d'au moins 5 % du biomarqueur normalisé de petite molécule à la suite d'une application avec un shampooing au sulfure de sélénium par comparaison avec un niveau de base de biomarqueur de petite molécule avant l'application.

10. Procédé selon l'une quelconque des revendications précédentes dans lequel il existe une augmentation d'au moins 5 % du biomarqueur normalisé de petite molécule à la suite d'une application avec un shampooing anti-pelliculaire par comparaison avec un niveau de base de biomarqueur de petite molécule avant l'application.

11. Procédé selon l'une quelconque des revendications précédentes dans lequel il existe une augmentation d'au moins 5 % du biomarqueur normalisé de petite molécule à la suite d'une application avec un shampooing à la pyrithione de zinc par comparaison avec un niveau de base de biomarqueur de petite molécule avant l'application.

12. Procédé selon l'une quelconque des revendications précédentes dans lequel il existe une augmentation d'au moins 5 % du biomarqueur normalisé de petite molécule à la suite d'une application avec un shampooing au sulfure de sélénium par comparaison avec un niveau de base de biomarqueur de petite molécule avant l'application.

13. Procédé selon l'une quelconque des revendications précédentes dans lequel il existe une amélioration d'au moins 5 % des pellicules par comparaison avec une population normale.

14. Procédé de mesure objective de la perception d'un symptôme de pellicules chez les mammifères, ledit procédé comprenant les étapes consistant à :
fournir un article adhésif qui a été appliqué à un épithélium d'un mammifère ; a été autorisé à adhérer à des cellules épithéliales ; et a été éliminé de l'épithélium du mammifère ;
préparer l'article adhésif en utilisant des procédés standard de laboratoire pour l'extraction ;
extraire un biomarqueur de petite molécule des cellules épithéliales mises en adhésion sur ledit article adhésif ;
mesurer un biomarqueur de petite molécule des cellules épithéliales mises en adhésion sur ledit article adhésif et ;
déterminer la quantité du biomarqueur de petite molécule dans les cellules épithéliales par comparaison avec un échantillon de base ; dans lequel le biomarqueur de petite molécule est l'uridine.

15. Procédé selon l'une quelconque des revendications précédentes dans lequel il existe une réduction du biomarqueur de petite molécule par rapport à un niveau de base qui est directement proportionnelle à une réduction d'une perception de symptôme de pellicules.
